(19) Europäisches Patentamt
European Patent Office
Office européen des brevets

(11) **EP 4 048 247 B1**

(12) **EUROPEAN PATENT SPECIFICATION**

(45) Date of publication and mention
of the grant of the patent:
**03.04.2024 Bulletin 2024/14**

(21) Application number: **20793689.9**

(22) Date of filing: **21.10.2020**

(51) International Patent Classification (IPC):
*A61K 31/137* (2006.01)    *A61K 9/70* (2006.01)
*A61P 37/00* (2006.01)    *A61K 47/06* (2006.01)
*A61K 47/10* (2017.01)

(52) Cooperative Patent Classification (CPC):
**A61K 31/137; A61K 9/7038; A61K 9/7053;
A61K 9/7061; A61K 9/7069; A61K 47/06;
A61K 47/10; A61P 37/00**

(86) International application number:
**PCT/EP2020/079549**

(87) International publication number:
**WO 2021/078761 (29.04.2021 Gazette 2021/17)**

(54) **TRANSDERMAL THERAPEUTIC SYSTEM FOR THE TRANSDERMAL ADMINISTRATION OF FINGOLIMOD**

TRANSDERMALES THERAPEUTISCHES SYSTEM ZUR TRANSDERMALEN VERABREICHUNG VON FINGOLIMOD

SYSTÈME THÉRAPEUTIQUE TRANSDERMIQUE POUR L'ADMINISTRATION TRANSDERMIQUE DE FINGOLIMOD

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.10.2019 EP 19205133**

(43) Date of publication of application:
**31.08.2022 Bulletin 2022/35**

(73) Proprietor: **LTS Lohmann Therapie-Systeme AG
56626 Andernach (DE)**

(72) Inventors:
• **HAMMES, Florian
56626 Andernach (DE)**

• **TOMELERI, Anja
56567 Neuwied (DE)**
• **KLEUDGEN, Tobias
56729 Ettringen (DE)**

(74) Representative: **Maiwald GmbH
Elisenhof
Elisenstraße 3
80335 München (DE)**

(56) References cited:
**US-A1- 2015 104 497    US-A1- 2018 028 461**

**Description**

## TECHNICAL FIELD OF THE INVENTION

The present invention relates to a transdermal therapeutic system (TTS) for the transdermal administration of fingolimod, processes of manufacture, and the TTS comprising fingolimod for use in a method of treatment.

## BACKGROUND OF THE INVENTION

The active ingredient fingolimod, 2-amino-2-[2-(4-octylphenyl)ethyl]propane-1,3-diol, is a sphingosine-1-phosphate receptor modulator. The immunomodulating drug sequesters lymphocytes in lymph nodes, preventing them from contributing to an autoimmune reaction.

Fingolimod-containing products are commercially available for treating patients with relapsing-remitting multiple sclerosis. The commercially available product GILENYA® contains 0.5 mg fingolimod hydrochloride in hard capsules for oral administration.

Several side effects have been associated with the product for oral administration. Fewer side effects in comparison to oral dosage forms are to be expected for transdermal therapeutic systems (TTS) for the transdermal administration of active agents. Furthermore, due to the simple mode of application of TTS, more convenience is accomplished for the patient. In particular, longer administration periods (e.g. one week) on the skin of human patients are beneficial for the compliance. Transdermal delivery systems comprising fingolimod are disclosed in US2018028461 A1. However, it is technically challenging to provide TTS with sufficient permeation rates of the active agent for time periods as needed and with the desired physical properties. Particularly in the case of fingolimod, which requires an enzymatic activation (phosphorylation) in the cell to cause the internalization of sphingosine-1-phosphate receptors, which sequesters lymphocytes in lymph nodes, the question is whether a TTS can deliver fingolimod to the enzymes for phosphorylation in an amount and a velocity sufficient for achieving a systemic effect.

Reports relating to substances which enhance the transdermal permeability of active agents are known, however, such transdermal permeation enhancer may even slow down the release of the active agent due to physical or chemical transformations of the active agent, and additionally may cause skin irritation and negatively affect the physical properties of the TTS (e.g. the adhesiveness and the cold flow properties). Since fingolimod, which has an amino-diol-structure, tends to react with functional groups, the provision of a TTS for the transdermal administration of fingolimod with sufficient permeation rates over an extended period of time is particularly challenging.

There is a need in the art for a fingolimod TTS that does not has the above mentioned problems. In particular, a need exists for a TTS for the transdermal administration of fingolimod, which provides an early onset of the therapeutic effect and a continuous delivery of fingolimod over an extended period of time, thereby improving patient compliance.

## OBJECTS AND SUMMARY OF THE INVENTION

It is an object of certain embodiments of the present invention to provide a TTS for the transdermal administration of fingolimod (e.g., fingolimod base).

It is a further object of certain embodiments of the present invention to provide a TTS for the transdermal administration of fingolimod (e.g., fingolimod base), which provides a permeation rate which is sufficient for achieving a therapeutically effective dose.

It is a further object of the present invention to provide a TTS for the transdermal administration of fingolimod (e.g., fingolimod base) with a high active-agent utilization, i.e. a TTS, which does not require a high excess amount of active agent to provide a sufficient release performance during an administration period.

It is an object of certain embodiments of the present invention to provide a TTS for the transdermal administration of fingolimod (e.g., fingolimod base), which provides an early onset of the permeation of fingolimod.

It is an object of certain embodiments of the present invention to provide a TTS for the transdermal administration of fingolimod (e.g., fingolimod base), which provides a sufficient permeation rate of fingolimod over the desired administration period (e.g., over 84 hours or 168 hours).

It is an object of certain embodiments of the present invention to provide a TTS for the transdermal administration of fingolimod (e.g., fingolimod base), which requires a relatively small amount of fingolimod contained therein, provides an early onset of the permeation of fingolimod and provides a sufficient permeation rate of fingolimod over the administration period (e.g., over 84 hours or 168 hours).

It is an object of certain embodiments of the present invention to provide a TTS for the transdermal administration of fingolimod that is easy to manufacture.

These objects and others are accomplished by the present invention which according to one aspect relates to a transdermal therapeutic system for the transdermal administration of fingolimod comprising a fingolimod-containing layer structure, the fingolimod-containing layer structure comprises a backing layer and a fingolimod-containing layer comprising a therapeutically effective amount of fingolimod (e.g. fingolimod base), at least one polymer, and dodecan-1-ol, wherein the weight ratio of dodecan-1-ol : fingolimod ranges from 1.5 : 1 to 5 : 1.

It has been found that the fingolimod (e.g. fingolimod base)-containing TTS according to the present invention provides an advantageous fingolimod delivery over an extended period of time with a short lag time of the permeation of fingolimod following the application of the TTS.

According to further aspects, the TTS according to the invention is for use in a method of treating an immune disorder, preferably multiple sclerosis. Thus, according to one aspect, the invention relates to the use of a TTS according to the invention for the manufacture of a medicament, preferably for the manufacture of a medicament for treating multiple sclerosis and a method of treating an immune disorder, preferably a method of treating multiple sclerosis.

According to yet another aspect, the invention relates to a method of manufacture of a TTS according to the invention comprising the steps of:

1) providing a fingolimod-containing coating composition comprising

a) fingolimod (e.g. fingolimod base),
b) at least one polymer,
c) dodecan-1-ol, and
d) optionally a solvent,

2) coating the fingolimod-containing coating composition onto a release liner in an amount to provide the desired area weight,
3) drying the coated fingolimod-containing coating composition to provide the fingolimod-containing layer,
4) laminating the fingolimod-containing layer to a backing layer to provide an fingolimod-containing layer structure,
5) optionally providing an additional skin contact layer by coating and drying an active agent-free coating composition or an active agent-containing coating composition according to steps 2 and 3, removing the release liner of the fingolimod-containing layer and laminating the adhesive side of the skin contact layer onto the adhesive side of the fingolimod-containing layer to provide an fingolimod-containing layer structure,
6) punching the individual systems from the fingolimod-containing layer structure,
7) optionally adhering to the individual systems an active agent-free self-adhesive layer structure comprising also a backing layer and an active agent-free pressure-sensitive adhesive layer and which is larger than the individual systems of the fingolimod-containing layer structure.

According to yet another aspect, the invention relates to the use of dodecan-1-ol in a transdermal therapeutic system for the transdermal administration of fingolimod for reducing the lag time of the permeation of fingolimod (e.g. fingolimod base).

According to a specific aspect, the invention relates to a transdermal therapeutic system for the transdermal administration of fingolimod comprising a fingolimod-containing layer structure, the fingolimod-containing layer structure comprising: A) a backing layer, and B) a fingolimod-containing layer, wherein the fingolimod-containing layer comprises a) a therapeutically effective amount of fingolimod (e.g. fingolimod base), b) at least one silicone acrylic hybrid pressure-sensitive adhesive, and c) dodecan-1-ol, wherein the weight ratio of dodecan-1-ol : fingolimod ranges from 1.5 : 1 to 5 : 1.

According to another specific aspect, the invention relates to a transdermal therapeutic system for the transdermal administration of fingolimod comprising a fingolimod-containing layer structure, the fingolimod-containing layer structure comprising: A) a backing layer, and B) a fingolimod-containing layer, wherein the fingolimod-containing layer comprises a) a therapeutically effective amount of fingolimod (e.g. fingolimod base), b) at least one polymer-based pressure-sensitive adhesive, c) dodecan-1-ol, and d) at least one cellulose derivative (e.g. ethyl cellulose), wherein the weight ratio of dodecan-1-ol : fingolimod ranges from 1.5 : 1 to 5 : 1.

According to yet another specific aspect, the invention relates to a transdermal therapeutic system for the transdermal administration of fingolimod comprising a fingolimod-containing layer structure, the fingolimod-containing layer structure comprising: A) a backing layer, and B) a fingolimod-containing layer; wherein the fingolimod-containing layer comprises a) a therapeutically effective amount of fingolimod (e.g. fingolimod base), b) at least one acrylate-based pressure-sensitive adhesive, and c) dodecan-1-ol, wherein the weight ratio of dodecan-1-ol:fingolimod ranges from 1.5 : 1 to 5 : 1, wherein the fingolimod-containing layer does not comprise a polymer selected from the group consisting of alkyl methacrylate copolymers, amino alkyl methacrylate copolymers, methacrylic acid copolymers, methacrylic ester copolymers, ammonioalkyl methacrylate copolymers, and polyvinylpyrrolidones, and wherein the fingolimod-containing layer does not comprise an organosulfur compound.

According to yet another specific aspect, the invention relates to a transdermal therapeutic system for the transdermal administration of fingolimod comprising a fingolimod-containing layer structure, the fingolimod-containing layer structure comprising: A) a backing layer, and B) a fingolimod-containing layer, wherein the fingolimod-containing layer comprises a) a therapeutically effective amount of fingolimod (e.g. fingolimod base), b) at least one pressure-sensitive adhesive based on polyisobutylenes or polysiloxanes, and c) dodecan-1-ol, wherein the weight ratio of dodecan-1-ol : fingolimod ranges from 1.5 : 1 to 5 : 1, wherein the fingolimod-containing layer does not comprise a polymer selected from the group consisting of alkyl methacrylate copolymers, amino alkyl methacrylate copolymers, methacrylic acid copolymers, methacrylic ester copolymers, ammonioalkyl methacrylate copolymers, and polyvinylpyrrolidones, and wherein the fingolimod-containing layer does not comprise an organosulfur compound.

**DEFINITIONS**

Within the meaning of this invention, the term "transdermal therapeutic system" (TTS) refers to a system by which the active agent (e.g. fingolimod) is administered to the systemic circulation *via* transdermal delivery and refers to the entire individual dosing unit that is applied, after removing an optionally present release liner, to the skin of a patient, and which comprises a therapeutically effective amount of active agent in an active agent-containing layer structure and optionally an additional adhesive overlay on top of the active agent-containing layer structure. The active agent-containing layer structure may be located on a release liner (a detachable protective layer), thus, the TTS may further comprise a release liner. Within the meaning of this invention, the term "TTS" in particular refers to systems providing transdermal delivery, excluding active delivery for example *via* iontophoresis or microporation. Transdermal therapeutic systems may also be referred to as transdermal drug delivery systems (TDDS) or transdermal delivery systems (TDS).

Within the meaning of this invention, the term "fingolimod-containing layer structure" refers to the layer structure containing a therapeutically effective amount of fingolimod and comprises a backing layer and at least one active agent-containing layer. Preferably, the fingolimod-containing layer structure is a fingolimod-containing self-adhesive layer structure.

Within the meaning of this invention, the term "therapeutically effective amount" refers to a quantity of active agent in the TTS which is, if administered by the TTS to a patient, sufficient to provide a treatment of an immune disorder, e.g. multiple sclerosis. A TTS usually contains more active in the system than is in fact provided to the skin and the systemic circulation. This excess amount of active agent is usually necessary to provide enough driving force for the delivery from the TTS to the systemic circulation.

Within the meaning of this invention, the terms "active", "active agent", and the like, as well as the term "fingolimod" refer to fingolimod in any pharmaceutically acceptable chemical and morphological form and physical state. These forms include without limitation fingolimod in its free base form, protonated or partially protonated fingolimod, fingolimod salts, and in particular acid addition salts formed by addition of an inorganic or organic acid such as fingolimod hydrochloride or fingolimod sulphate, phosphate, tartrate, maleinate, oxalate, acetate, lactate, solvates, hydrates, clathrates, complexes and so on, as well as fingolimod in the form of particles which may be micronized, crystalline and/or amorphous, and any mixtures of the aforementioned forms. The fingolimod, where contained in a medium such as a solvent, may be dissolved or dispersed or in part dissolved and in part dispersed. In the present invention, fingolimod base is preferably dissolved or dispersed in the solvent of a polymer to form a fingolimod-containing coating composition and is contained in the dried matrix layer in dissolved or dispersed form, or in part dissolved and in part dispersed form.

When fingolimod is mentioned to be used in a particular form in the manufacture of the TTS, this does not exclude interactions between this form of fingolimod and other ingredients of the fingolimod-containing layer structure, e.g. salt formation or complexation, in the final TTS. This means that, even if fingolimod is included in its free base form, it may be present in the final TTS in protonated or partially protonated form or in the form of an acid addition salt, or, if it is included in the form of a salt, parts of it may be present as free base in the final TTS. Unless otherwise indicated, in particular the amount of fingolimod in the layer structure relates to the amount of fingolimod included in the TTS during manufacture of the TTS and is calculated based on fingolimod in the form of the free base. E.g., when a) 0.1 mmol (equal to 30.75 mg) fingolimod base or b) 0.1 mmol (equal to 34.39 mg) fingolimod hydrochloride is included in the TTS during manufacture, the amount of fingolimod in the layer structure is, within the meaning of the invention, in both cases 30.75 mg, i.e. 0.1 mmol.

The fingolimod starting material included in the TTS during manufacture of the TTS may be in the form of particles. Fingolimod may e.g. be present in the active agent-containing layer structure in the form of particles, which are preferably homogeneously dispersed within the active agent-containing layer structure.

Within the meaning of this invention, the term "particles" refers to a solid, particulate material comprising individual particles, the dimensions of which are negligible compared to the material. In particular, the particles are solid, including plastic/deformable solids, including amorphous and crystalline materials.

Within the meaning of this invention, the term "dispersing" refers to a step or a combination of steps wherein a starting material (e.g. fingolimod) is not dissolved or not completely dissolved. Dispersing in the sense of the invention comprises the dissolution of a part of the starting material (e.g. fingolimod particles), depending on the solubility of the starting material (e.g. the solubility of fingolimod in the coating composition).

There are two main types of TTS for active agent delivery, i.e. matrix-type TTS and reservoir-type TTS. The release of the active agent in a matrix-type TTS is mainly controlled by the matrix including the active agent itself. In contrast thereto, a reservoir-type TTS typically needs a rate-controlling membrane controlling the release of the active agent. In principle, also a matrix-type TTS may contain a rate-controlling membrane. However, matrix-type TTS are advantageous in that, compared to reservoir-type TTS, usually no rate determining membranes are necessary and no dose dumping can occur due to membrane rupture. In summary, matrix-type transdermal therapeutic systems (TTS) are less complex in manufacture and easy and convenient to use by patients.

Within the meaning of this invention, "matrix-type TTS" refers to a system or structure wherein the active is homogeneously dissolved and/or dispersed within a polymeric carrier, i.e. the matrix, which forms with the active agent and optionally remaining ingredients a matrix layer. In such a system, the matrix layer controls the release of the active agent from the TTS. Preferably, the matrix layer has sufficient cohesion to be self-supporting so that no sealing between other layers is required. Accordingly, the active agent-containing layer may in one embodiment of the invention be an active agent-containing matrix layer, wherein the active agent is homogeneously distributed within a polymer matrix. In certain embodiments, the active agent-containing matrix layer may comprise two active agent-containing matrix layers, which may be laminated together. Matrix-type TTS may in particular be in the form of a "drug-in-adhesive"-type TTS referring to a system wherein the active is homogeneously dissolved and/or dispersed within a pressure-sensitive adhesive matrix. In this connection, the active agent-containing matrix layer may also be referred to as active agent-containing pressure sensitive adhesive layer or active agent-containing pressure sensitive adhesive matrix layer. A TTS comprising the active agent dissolved and/or dispersed within a polymeric gel, e.g. a hydrogel, is also considered to be of matrix-type in accordance with present invention.

TTS with a liquid active agent-containing reservoir are referred to by the term "reservoir-type TTS". In such a system, the release of the active agent is preferably controlled by a rate-controlling membrane. In particular, the reservoir is sealed between the backing layer and the rate-controlling membrane. Accordingly, the active agent-containing layer may in one embodiment be an active agent-containing reservoir layer, which preferably comprises a liquid reservoir comprising the active agent. Furthermore, the reservoir-type TTS typically additionally comprises a skin contact layer, wherein the reservoir layer and the skin contact layer may be separated by the rate-controlling membrane. In the reservoir layer, the active agent is preferably dissolved in a solvent such as ethanol or water or in silicone oil. The skin contact layer typically has adhesive properties.

Reservoir-type TTS are not to be understood as being of matrix-type within the meaning of the invention. However, microreservoir TTS (biphasic systems having deposits (e.g. spheres, droplets) of an inner active-containing phase dispersed in an outer polymer phase), considered in the art to be a mixed from of a matrix-type TTS and a reservoir-type TTS that differ from a homogeneous single phase matrix-type TTS and a reservoir-type TTS in the concept of drug transport and drug delivery, are considered to be of matrix-type within the meaning of the invention. The sizes of microreservoir droplets can be determined by an optical microscopic measurement (for example by Leica MZ16 including a camera, for example Leica DSC320) by taking pictures of the microreservoirs at different positions at an enhancement factor between 10 and 400 times, depending on the required limit of detection. By using imaging analysis software, the sizes of the microreservoirs can be determined.

Within the meaning of this invention, the term "active agent-containing layer" refers to a layer containing the active agent and providing the area of release. The term covers active agent-containing matrix layers and active agent-containing reservoir layers. If the active agent-containing layer is an active agent-containing matrix layer, said layer is present in a matrix-type TTS. If the polymer is a pressure-sensitive adhesive, the matrix layer may also represent the adhesive layer of the TTS, so that no additional skin contact layer is present. Alternatively, an additional skin contact layer may be present as adhesive layer, and/or an adhesive overlay is provided. The additional skin contact layer is typically manufactured such that it is active agent-free. However, due to the concentration gradient, the active agent will migrate from the matrix layer to the additional skin contact layer over time, until equilibrium is reached. The additional skin contact layer may be present on the active agent-containing matrix layer or separated from the active agent-containing matrix layer by a membrane, preferably a rate controlling membrane. Preferably, the active agent-containing matrix layer has sufficient adhesive properties, so that no additional skin contact layer is present. If the active agent-containing layer is an active agent-containing reservoir layer, said layer is present in a reservoir-type TTS, and the layer comprises the active agent in a liquid reservoir. In addition, an additional skin contact layer is preferably present, in order to provide adhesive properties. Preferably, a rate-controlling membrane separates the reservoir layer from the additional skin contact layer. The additional skin contact layer can be manufactured such that it is active agent-free or active agent-containing. If the additional skin contact layer is free of active agent the active agent will migrate, due to the concentration gradient, from the reservoir layer to the skin contact layer over time, until equilibrium is reached. Additionally an adhesive overlay may be provided.

As used herein, the active agent-containing layer is preferably an active agent-containing matrix layer, and it is referred to the final solidified layer. Preferably, an active agent-containing matrix layer is obtained after coating and drying the solvent-containing coating composition as described herein. Alternatively an active-agent containing matrix layer is obtained after melt-coating and cooling. The active agent-containing matrix layer may also be manufactured by laminating two or more such solidified layers (e.g. dried or cooled layers) of the same composition to provide the desired area weight. The matrix layer may be self-adhesive (in the form of a pressure sensitive adhesive matrix layer), or the TTS may comprise an additional skin contact layer of a pressure sensitive adhesive for providing sufficient tack. Preferably, the matrix layer is a pressure sensitive adhesive matrix layer. Optionally, an adhesive overlay may be present. A matrix layer according to the present invention does not contain a non-volatile solvent in an amount of more than 40 % by weight based on the matrix layer. In certain embodiments of the invention, the active agent-containing matrix layer contains a non-volatile solvent in an amount of from about 5 % to about 20 % by weight based on the active agent-containing matrix layer. Preferably, the active agent-containing matrix layer contains no non-volatile solvents.

Within the meaning of this invention, the term "pressure-sensitive adhesive" (also abbreviated as "PSA") refers to a material that in particular adheres with finger pressure, is permanently tacky, exerts a strong holding force and should be removable from smooth surfaces without leaving a residue. A pressure sensitive adhesive layer, when in contact with the skin, is "self-adhesive", i.e. provides adhesion to the skin so that typically no further aid for fixation on the skin is needed. A "self-adhesive" layer structure includes a pressure sensitive adhesive layer for skin contact which may be provided in the form of a pressure sensitive adhesive matrix layer or in the form of an additional layer, i.e. a pressure sensitive adhesive skin contact layer. An adhesive overlay may still be employed to advance adhesion. The pressure-sensitive adhesive properties of a pressure-sensitive adhesive depend on the polymer or polymer composition used.

Within the meaning of this invention, the term "silicone acrylic hybrid polymer" refers to a polymerization product including repeating units of a silicone sub-species and an acrylate-sub species. The silicone acrylic hybrid polymer thus comprises a silicone phase and an acrylic phase. The term "silicone acrylic hybrid" is intended to denote more than a simple blend of a silicone-based sub-species and an acrylate-based sub-species. Instead, the term denotes a polymerized hybrid species that includes silicone-based sub-species and acrylate-based sub-species that have been polymerized together. The silicone acrylic hybrid polymer may also be referred to as a "silicone acrylate hybrid polymer" as the terms acrylate and acrylic are generally used interchangeably in the context of the hybrid polymers used in the present invention.

Within the meaning of this invention, the term "silicone acrylic hybrid pressure-sensitive adhesive" refers to a silicone acrylic hybrid polymer in the form of a pressure-sensitive adhesive. Silicone acrylic hybrid pressure-sensitive adhesives are described, for example, in EP 2 599 847 and WO 2016/130408. Examples of silicone acrylic hybrid pressure-sensitive adhesives include the PSA series 7-6100 and 7-6300 manufactured and supplied in n-heptane or ethyl acetate by Dow Corning (7-610X and 7-630X; X=1 n-heptane-based / X=2 ethyl acetate-based). It was found that, depending on the solvent in which the silicone acrylic hybrid PSA is supplied, the arrangement of the silicone phase and the acrylic phase providing a silicone or acrylic continuous external phase and a corresponding discontinuous internal phase is different. If the silicone acrylic hybrid PSA is supplied in n-heptane, the composition contains a continuous, silicone external phase and a discontinuous, acrylic internal phase. If the silicone acrylic hybrid PSA composition is supplied in ethyl acetate, the composition contains a continuous, acrylic external phase and a discontinuous, silicone internal phase.

Within the meaning of this invention, the term "silicon-containing pressure-sensitive adhesive composition comprising acrylate or methacrylate functionality" comprises the condensation reaction product of a silicone resin, a silicone polymer, and a silicon-containing capping agent which provides said acrylate or methacrylate functionality. It is to be understood that the silicon-containing pressure-sensitive adhesive composition comprising acrylate or methacrylate functionality can include only acrylate functionality, only methacrylate functionality, or both acrylate functionality and methacrylate functionality.

As used herein, an active agent-containing matrix layer is a layer containing the active agent dissolved or dispersed in at least one polymer, or containing the active agent dissolved in a solvent to form an active agent-solvent mixture that is dispersed in the form of deposits (in particular droplets) in at least one polymer. Preferably, the at least one polymer is a polymer-based pressure-sensitive adhesive (e.g. a silicone pressure-sensitive adhesive or a silicone acrylic hybrid pressure-sensitive adhesive). Within the meaning of this invention, the term "pressure-sensitive adhesive layer" refers to a pressure-sensitive adhesive layer obtained from a solvent-containing adhesive coating composition after coating on a film and evaporating the solvents.

Within the meaning of this invention, the term "skin contact layer" refers to the layer included in the active agent-containing layer structure to be in direct contact with the skin of the patient during administration. This may be the active agent-containing layer. When the TTS comprises an additional skin contact layer, the other layers of the active agent-containing layer structure do not contact the skin and do not necessarily have self-adhesive properties. As outlined above, an additional skin contact layer attached to the active agent-containing layer may over time absorb parts of the active agent. An additional skin contact layer may be used to enhance adherence. The sizes of an additional skin contact layer and the active agent-containing layer are usually coextensive and correspond to the area of release. However, the area of the additional skin contact layer may also be greater than the area of the active agent-containing layer. In such a case, the area of release still refers to the area of the active agent-containing layer.

Within the meaning of this invention, the term "area weight" refers to the dry weight of a specific layer, e.g. of the matrix layer, provided in g/m$^2$. The area weight values are subject to a tolerance of ± 10 %, preferably ± 7.5 %, due to manufacturing variability.

If not indicated otherwise "%" refers to % by weight.

Within the meaning of this invention, the term "polymer" refers to any substance consisting of so-called repeating units obtained by polymerizing one or more monomers, and includes homopolymers which consist of one type of monomer and copolymers which consist of two or more types of monomers. Polymers may be of any architecture such as linear polymers, star polymer, comb polymers, brush polymers, of any monomer arrangements in case of copolymers, e.g. alternating, statistical, block copolymers, or graft polymers. The minimum molecular weight varies depending on the polymer type and is known to the skilled person. Polymers may e.g. have a molecular weight above 2000, preferably above 5000 and more preferably above 10,000 Dalton. Correspondingly, compounds with a molecular weight below 2000, preferably below 5000 or more preferably below 10,000 Dalton are usually referred to as oligomers.

Within the meaning of this invention, the term "cross-linking agent" refers to a substance which is able to cross-link functional groups contained within the polymer.

Within the meaning of this invention, the term "adhesive overlay" refers to a self-adhesive layer structure that is free of active agent and larger in area than the active agent-containing structure and provides additional area adhering to the skin, but no area of release of the active agent. It enhances thereby the overall adhesive properties of the TTS. The adhesive overlay comprises a backing layer that may provide occlusive or non-occlusive properties and an adhesive layer. Preferably, the backing layer of the adhesive overlay provides non-occlusive properties.

Within the meaning of this invention, the term "backing layer" refers to a layer which supports the active agent-containing layer or forms the backing of the adhesive overlay. At least one backing layer in the TTS and usually the backing layer of the active agent-containing layer is substantially impermeable to the active agent contained in the layer during the period of storage and administration and thus prevents active loss or cross-contamination in accordance with regulatory requirements. Preferably, the backing layer is also occlusive, meaning substantially impermeable to water and water-vapor. Suitable materials for a backing layer include polyethylene terephthalate (PET), polyethylene (PE), ethylene vinyl acetate-copolymer (EVA), polyurethanes, and mixtures thereof. Suitable backing layers are thus for example PET laminates, EVA-PET laminates and PE-PET laminates. Also suitable are woven or non-woven backing materials.

The TTS according to the present invention can be characterized by certain parameters as measured in an *in vitro* skin permeation test.

Where not otherwise indicated, the *in vitro* permeation test is performed with dermatomed split-thickness human skin with a thickness of 500 μm and an intact epidermis, and with phosphate buffer pH 5.5 as receptor medium (32 °C with 0.1 % Methyl-β-Cyclodextrine and 0.1 % saline azide). The amount of active permeated into the receptor medium is determined in regular intervals using a validated HPLC method with a UV photometric detector by taking a sample volume. The receptor medium is completely or in part replaced by fresh medium when taking the sample volume, and the measured amount of active permeated relates to the amount permeated between the two last sampling points and not the total amount permeated so far.

Thus, within the meaning of this invention, the parameter "permeated amount" is provided in μg/cm$^2$ and relates to the amount of active permeated in a sample interval at certain elapsed time. E.g., in an *in vitro* permeation test as described above, wherein the amount of active permeated into the receptor medium has been e.g. measured at hours 0, 8, 24, 36, 48, 72, 104, 144, and 168, the "permeated amount" of active can be given e.g. for the sample interval from hour 36 to hour 48 and corresponds to the measurement at hour 48, wherein the receptor medium has been exchanged completely at hour 36.

The permeated amount can also be given as a "cumulative permeated amount", corresponding to the cumulated amount of active permeated at a certain point in time. E.g., in an *in vitro* permeation test as described above, wherein the amount of active permeated into the receptor medium has been e.g. measured at hours 0, 8, 24, 36, 48, 72, 104, 144, and 168, the "cumulative permeated amount" of active at hour 48 corresponds to the sum of the permeated amounts from hour 0 to hour 8, hour 8 to hour 24, hour 24 to hour 36, and hour 36 to hour 48.

Within the meaning of this invention, the parameter "skin permeation rate" for a certain sample interval at certain elapsed time is provided in μg/cm$^2$-hr and is calculated from the permeated amount in said sample interval as measured by *in vitro* permeation test as described above in μg/cm$^2$, divided by the hours of said sample interval. E.g. the skin permeation rate in an *in vitro* permeation test as described above, wherein the amount of active permeated into the receptor medium has been e.g. measured at hours 0, 8, 24, 36, 48, 72, 104, 144, and 168, the "skin permeation rate" at hour 48 is calculated as the permeated amount in the sample interval from hour 36 to hour 48 divided by 12 hours.

A "cumulative skin permeation rate" can be calculated from the respective cumulative permeated amount by dividing the cumulative permeated amount by the elapsed time. E.g. in an *in vitro* permeation test as described above, wherein the amount of active permeated into the receptor medium has been e.g. measured at hours 0, 8, 24, 32, 48, 72, 104, 144, and 168, the "cumulative skin permeation rate" at hour 48 is calculated as the cumulative permeated amount at hour 48 (see above) divided by 48 hours, unless indicated otherwise.

Within the meaning of this invention, the term "active agent utilization" refers to the cumulative permeated amount after a certain elapsed time, e.g. after 72 hours, divided by the initial loading of the active agent.

Within the meaning of this invention, the above parameters "permeated amount" and "skin permeation rate" (as well as "cumulative permeated amount" and "cumulative skin permeation rate") refer to mean values calculated from at least 3 *in vitro* permeation test experiments. Where not otherwise indicated, the standard deviation (SD) of these mean values refer to a corrected sample standard deviation, calculated using the formula:

$$SD = \sqrt{\frac{1}{n-1}\sum_{i=1}^{n}(x_i - \overline{x})^2}$$

wherein n is the sample size, $\{x_1, x_2, ... x_n\}$ are the observed values and $\overline{x}$ is the mean value of the observed values.

The TTS according to the present invention can also be characterized by certain parameters as measured in an *in vivo* clinical study.

Within the meaning of this invention, the parameter "mean release rate" refers to the mean release rate in $\mu$g/hr or in mg/day over the period of administration (e.g., 1 to 7 days) by which the active agent is released through the human skin into the systemic circulation and is based on the AUC obtained over said period of administration in a clinical study.

Within the meaning of this invention, the term "extended period of time" relates to a period of at least or about 72 hours (3 days), about 84 hours (3.5 days), at least or about 96 hours (4 days), or about 168 hours (7 days).

Within the meaning of this invention, the term "room temperature" refers to the unmodified temperature found indoors in the laboratory where the experiments are conducted and usually lies within 15 to 35 °C, preferably about 18 to 25 °C.

Within the meaning of this invention, the term "patient" refers to a subject who has presented a clinical manifestation of a particular symptom or symptoms suggesting the need for treatment, who is treated preventatively or prophylactically for a condition, or who has been diagnosed with a condition to be treated.

Within the meaning of this invention the term "pharmacokinetic parameters" refers to parameters describing the blood curve, e.g. $C_{max}$, Ct and $AUC_{t1-t2}$ obtained in a clinical study, e.g. by single-dose, multi-dose or steady state administration of the active agent-containing TTS, e.g. the fingolimod-containing TTS to healthy human subjects. The pharmacokinetic parameters of the individual subjects are summarized using arithmetic and geometric means, e.g. a mean $C_{max}$, a mean AUCt and a mean AUCINF, and additional statistics such as the respective standard deviations and standard errors, the minimum value, the maximum value, and the middle value when the list of values is ranked (Median). In the context of the present invention, pharmacokinetic parameters, e.g. the $C_{max}$, Ct and $AUC_{t1-t2}$ refer to geometric mean values if not indicated otherwise. It cannot be precluded that the absolute mean values obtained for a certain TTS in a clinical study vary to a certain extent from study to study. To allow a comparison of absolute mean values between studies, a reference formulation, e.g. in the future any product based on the invention, may be used as internal standard. A comparison of the AUC per area of release of the respective reference product in the earlier and later study can be used to obtain a correction factor to take into account differences from study to study.

Clinical studies according to the present invention refer to studies performed in full compliance with the International Conference for Harmonization of Clinical Trials (ICH) and all applicable local Good Clinical Practices (GCP) and regulations.

Within the meaning of this invention, the term "subject population" refers to at least five, preferably at least ten individual subjects.

Within the meaning of this invention, the term "geometric mean" refers to the mean of the log transformed data back-transformed to the original scale.

Within the meaning of this invention, the term "arithmetic mean" refers to the sum of all values of observation divided by the total number of observations.

Within the meaning of this invention, the parameter "AUC" corresponds to the area under the concentration-time curve. The AUC value is proportional to the amount of active agent absorbed into the blood circulation in total and is hence a measure for the bioavailability.

Within the meaning of this invention, the parameter "$AUC_{t1-t2}$" is provided in (ng / ml) hr and relates to the area under the concentration-time curve from hour t1 to t2 and is calculated by the linear trapezoidal method, unless otherwise indicated. Other calculation methods are e.g. the logarithmic and linear log trapezoidal method.

Within the meaning of this invention, the parameter "$C_{max}$" is provided in (ng / ml) and relates to the maximum observed blood concentration of the active agent.

Within the meaning of this invention, the parameter "Ct" is provided in (ng / ml) and relates to the blood concentration of the active agent observed at hour t.

Within the meaning of this invention, the parameter "$t_{max}$" is provided in hr and relates to the time point at which the $C_{max}$ value is reached. In other words, $t_{max}$ is the time point of the maximum observed concentration.

Within the meaning of this invention, the term "coating composition" refers to a composition comprising all components of the matrix layer in a solvent, which may be coated onto the backing layer or release liner to form the matrix layer upon drying.

Within the meaning of this invention, the term "pressure sensitive adhesive composition" refers to a pressure sensitive adhesive at least in mixture with a solvent (e.g. n-heptane or ethyl acetate).

Within the meaning of this invention, the term "dissolve" refers to the process of obtaining a solution, which is clear and does not contain any particles, as visible to the naked eye.

Within the meaning of this invention, the term "solvent" refers to any liquid substance, which preferably is a volatile organic liquid such as methanol, ethanol, isopropanol, acetone, ethyl acetate, methylene chloride, hexane, n-heptane, toluene and mixtures thereof.

## BRIEF DESCRIPTION OF THE DRAWINGS

Fig. 1a depicts the cumulative permeated amount of fingolimod for TTS prepared according to Example 1, Comparative Example A, Example 2 and Comparative Example B over a time interval of 168 hours.

Fig. 1b depicts the skin permeation rate of fingolimod for TTS prepared according to Example 1, Comparative Example A, Example 2 and Comparative Example B over a time interval of 168 hours.

Fig. 2a depicts the cumulative permeated amount of fingolimod for TTS prepared according to Example 3, Comparative Example C, Example 4 and Comparative Example D over a time interval of 168 hours.

Fig. 2b depicts the skin permeation rate of fingolimod for TTS prepared according to Example 3, Comparative Example C, Example 4 and Comparative Example D over a time interval of 168 hours.

Fig. 3a depicts the cumulative permeated amount of fingolimod for TTS prepared according to Example 4, Comparative Example D, Example 5 and Comparative Example E over a time interval of 168 hours.

Fig. 3b depicts the skin permeation rate of fingolimod for TTS prepared according to Example 4, Comparative Example D, Example 5 and Comparative Example E over a time interval of 168 hours.

Fig. 4a depicts the cumulative permeated amount of fingolimod for TTS prepared according to Example 1, Comparative Example A, and Comparative Example F over a time interval of 168 hours.

Fig. 4b depicts the skin permeation rate of fingolimod for TTS prepared according to Example 1, Comparative Example A, and Comparative Example F over a time interval of 168 hours.

Fig. 5a depicts the cumulative permeated amount of fingolimod for TTS prepared according to Example 7-1 and Example 7-2 over a time interval of 168 hours.

Fig. 5b depicts the skin permeation rate of fingolimod for TTS prepared according to Example 7-1 and Example 7-2 over a time interval of 168 hours.

Fig. 6 depicts the concentrations of fingolimod and fingolimod phosphate in minipig whole blood after daily oral administration of Gilenya™ capsules 0.5 mg fingolimod over a time interval of 168 hours.

Fig. 7 depicts the concentrations of fingolimod in minipig whole blood after application of TTS prepared according to Examples 9-1 to 9-4 over a time interval of 168 hours.

Fig. 8 depicts the concentrations of fingolimod phosphate in minipig whole blood after application of TTS prepared according to Examples 9-1 to 9-4 over a time interval of 168 hours.

## DETAILED DESCRIPTION

## TTS STRUCTURE

The present invention relates to a transdermal therapeutic system for the transdermal administration of fingolimod comprising a fingolimod-containing layer structure.

The fingolimod-containing layer structure according to the invention comprises A) a backing layer and B) a fingolimod-containing layer comprising a therapeutically effective amount of fingolimod, at least one polymer, and dodecan-1-ol. The fingolimod-containing layer structure is preferably a fingolimod-containing self-adhesive layer structure.

The backing layer is preferably substantially fingolimod-impermeable. Furthermore, it is preferred that the backing layer is occlusive as outlined above.

The fingolimod-containing layer may be directly attached to the backing layer, so that no further layer between the backing layer and the fingolimod-containing layer is present.

The TTS according to the present invention may be a matrix-type TTS or a reservoir-type TTS, and preferably is a matrix-type TTS.

The fingolimod-containing layer structure according to the invention is normally located on a detachable protective layer (release liner), from which it is removed immediately before application to the surface of the patient's skin. Thus, the TTS may further comprise a release liner. A TTS protected this way is usually stored in a blister pack or a seam-sealed pouch. The packaging may be child resistant and/or senior friendly.

In a preferred embodiment of the present invention, the fingolimod-containing layer is a fingolimod-containing pressure sensitive adhesive layer and represents the skin contact layer. That is, the fingolimod-containing layer structure does not comprise an additional skin contact layer attached to the fingolimod-containing layer. In this connection, the fingolimod-containing layer is preferably a fingolimod-containing matrix layer, which is self-adhesive. The self-adhesive properties of the fingolimod-containing layer structure are preferably provided by the polymer. Thus, in a preferred embodiment of the invention, the at least one polymer is a pressure sensitive adhesive. Further details regarding the fingolimod-containing layer and the at least one polymer according to the invention are provided further below.

In another embodiment of the present invention, the fingolimod-containing layer structure further comprises an additional skin contact layer. The skin contact layer is preferably self-adhesive and provides adhesive properties. Thus, in one embodiment of the present invention, the fingolimod-containing layer structure further comprises C) a skin contact layer on the fingolimod-containing layer. In this connection, the additional skin contact layer may also contain at least one polymer, which may be the same polymer as the at least one polymer contained in the fingolimod-containing layer or a different polymer. For example, when the additional skin contact layer comprises a pressure-sensitive adhesive based on polysiloxanes, the fingolimod-containing layer may comprises the same pressure-sensitive adhesive based on polysiloxanes, or a different pressure-sensitive adhesive based on polysiloxanes or a different non-hybrid polymer or a hybrid polymer. The additional skin contact layer is preferably obtainable by coating and drying an adhesive coating composition.

In certain embodiments of the invention, wherein the fingolimod-containing layer structure comprises an additional skin contact layer, the additional skin contact layer has an area weight of from about 10 to about 160 $g/m^2$, from about 10 to about 100 $g/m^2$, or from about 10 to about 60 $g/m^2$. The total amount of polymer contained in the skin contact layer may range from about 40 % to about 100 % by weight, preferably from about 50 % to about 100 % by weight, more preferably from about 60 % to about 100 % by weight based on the skin contact layer. The skin contact layer may comprise an active agent. The active agent may be fingolimod, as well. The active agent in the skin contact layer may also be an additional active agent reasonable for an administration together with fingolimod. In a preferred embodiment, the skin contact layer is free of active agent, that is, is prepared without the addition of an active agent.

According to certain embodiments of the invention, the TTS may further comprise an adhesive overlay. This adhesive overlay is in particular larger in area than the fingolimod-containing structure and is attached thereto for enhancing the adhesive properties of the overall transdermal therapeutic system. Said adhesive overlay comprises a backing layer and an adhesive layer. The adhesive overlay provides additional area adhering to the skin but does not add to the area of release of the fingolimod. The adhesive overlay comprises a self-adhesive polymer or a self-adhesive polymer mixture selected from the group consisting of silicone acrylic hybrid polymers, acrylic polymers, polysiloxanes, polyisobutylenes, and mixtures thereof, which may be identical to or different from any polymer or polymer mixture included in the fingolimod-containing layer structure. In one embodiment, the TTS is free of an adhesive overlay on top of the fingolimod-containing layer structure.

Depending on the dosage, the area of release of the TTS ranges from about 1 $cm^2$ to about 50 $cm^2$, preferably from about 1 $cm^2$ to less than 50 $cm^2$.

The TTS according to the invention may further comprise one or more anti-oxidants. The anti-oxidants may be contained in the fingolimod-containing layer or in an additional skin contact layer or in both the fingolimod-containing layer and the additional skin contact layer. Suitable anti-oxidants are sodium metabisulfite, ascorbyl palmitate, tocopherol and esters thereof, ascorbic acid, butylhydroxytoluene, butylhydroxyanisole or propyl gallate, preferably butylhydroxytoluene, ascorbyl palmitate and tocopherol. The anti-oxidants may be conveniently present in the fingolimod-containing layer, preferably in an amount of from about 0.001 to about 1.0 % of the fingolimod-containing layer, more preferably in an amount of from about 0.02 to about 0.5 % of the fingolimod-containing layer.

The TTS according to the invention may further comprise in addition to the above mentioned ingredients at least one excipient or additive, for example from the group of cross-linking agents, solubilizers, fillers, tackifiers, film-forming agents, plasticizers, stabilizers, softeners, substances for skincare, permeation enhancers, pH regulators, and preservatives. In general, it is preferred according to the invention that no additional excipients or additives are required. Thus, the TTS has a composition of low complexity. In certain embodiments, no further additive (e.g. a transdermal permeation enhancer) is present in the TTS.

## FINGOLIMOD-CONTAINING LAYER

As outlined in more detail above, the TTS according to the present invention comprises a fingolimod-containing layer structure comprising a fingolimod-containing layer. The fingolimod-containing layer according to the invention comprises a therapeutically effective amount of fingolimod, at least one polymer, and dodecan-1-ol. The weight ratio of dodecan-1-ol to fingolimod in the fingolimod-containing layer ranges from 1.5 to 1 - 5 to 1.

According to certain embodiments, the fingolimod-containing layer contains dodecan-1-ol and fingolimod in a weight ratio of dodecan-1-ol to fingolimod of 1.5 to 1 - 4 to 1, of 1.5 to 1 - 2.5 to 1, of 3 to 1 - 4 to 1, of about 2.0 to 1, or about 3.3 to 1.

The fingolimod-containing layer may be a fingolimod-containing matrix layer or a fingolimod-containing reservoir layer. It is preferred that the fingolimod-containing layer is a fingolimod-containing matrix layer, which comprises fingolimod homogeneously dispersed or dissolved in the polymer matrix. In another preferred embodiment, the fingolimod-containing layer is a fingolimod-containing biphasic matrix layer, which comprises an inner phase comprising the therapeutically effective amount of fingolimod, and an outer phase comprising the at least one polymer, wherein the inner phase forms dispersed deposits in the outer phase. The content of the inner phase in the biphasic matrix layer may be from about 5 % to 40 % by volume based on the volume of the biphasic matrix layer.

According to certain preferred embodiments, the fingolimod-containing layer is a self-adhesive fingolimod-containing matrix layer.

In a certain embodiment, the fingolimod-containing layer is obtainable by coating and drying a fingolimod-containing coating composition that comprises the fingolimod in the form of the free base, preferably by coating and drying a fingolimod-containing coating composition, which comprises the at least one polymer, and the dodecan-1-ol and the therapeutically effective amount of fingolimod in a weight ratio of dodecan-1-ol : fingolimod of from 1.5 : 1 to 5 : 1.

According to certain embodiments, the fingolimod-containing layer has an area weight of from about 50 to about 200 g/m$^2$, preferably from about 60 to about 180 g/m$^2$, more preferably from about 80 to about 160 g/m$^2$, of about 100 g/m$^2$, or of about 150 g/m$^2$.

According to certain embodiments, the fingolimod-containing layer contains dodecan-1-ol in an amount of from 2 % to 40 %, preferably from 2 % to 30 %, more preferably from 4 % to 20 %, of about 10 %, or about 15 % by weight based on the fingolimod-containing layer.

According to certain embodiments, the fingolimod-containing layer contains the fingolimod in an amount of from 1 % to 20 %, preferably from 1 % to 15 %, more preferably from 2 % to 10 %, of about 3 %, or about 7.5 % by weight based on the fingolimod-containing layer.

In certain embodiments, the fingolimod in the fingolimod-containing layer may be included in the form of a pharmaceutically acceptable chemical and morphological form and physical state, such as a pharmaceutically acceptable salt thereof. In one embodiment, the fingolimod-containing layer comprises a pharmaceutically acceptable salt of fingolimod, such as fingolimod hydrochloride. However, it is preferred according to the invention that the fingolimod in the fingolimod-containing layer is included in the form of the free base.

According to certain embodiments, the fingolimod-containing layer contains the at least one polymer in an amount of from about 40 % to about 99 % by weight, preferably of from about 50 % to about 99 % by weight, more preferably of from about 60 % to about 99 % by weight based on the fingolimod-containing layer. The at least one polymer is preferably selected from the group consisting of a silicone acrylic hybrid polymer, a polymer based on polysiloxanes, a polymer based on polyisobutylenes, and an acrylate polymer. Further details regarding the at least one polymer according to the invention are provided further below.

In one embodiment, the fingolimod-containing layer comprises a further polymer selected from the group consisting of a silicone acrylic hybrid polymer, a polymer based on polysiloxanes, a polymer based on polyisobutylenes, and an acrylate polymer. The fingolimod-containing layer thus may comprise a blend of at least two polymers which are characterized by different chemical or physical properties. In a particular embodiment, the fingolimod-containing layer comprises a blend of at least two polyisobutylenes which are characterized by different physical properties.

In certain embodiments, the fingolimod-containing layer further comprises an auxiliary polymer. The auxiliary polymer may be contained in an amount of from about 0.5 % to about 20 %, from about 0.5 % to about 10 %, or from about 1 % to about 5 % by weight based on the fingolimod-containing layer. The auxiliary polymer is preferably selected from the group consisting of alkyl methacrylate copolymers, amino alkyl methacrylate copolymers, methacrylic acid copolymers, methacrylic ester copolymers, ammonioalkyl methacrylate copolymers, polyvinylpyrrolidones, vinylpyrrolidone-vinyl acetate copolymers, polyvinyl caprolactam-polyvinyl acetate-polyethylene glycol copolymer, cellulose derivatives, and mixtures thereof. In one embodiment, the auxiliary polymer is a cellulose derivative, preferably ethyl cellulose.

In certain other embodiments, the fingolimod-containing layer does not comprise a polyvinylpyrrolidone, preferably the fingolimod-containing layer does not comprise a polymer selected from the group consisting of alkyl methacrylate copolymers, amino alkyl methacrylate copolymers, methacrylic acid copolymers, methacrylic ester copolymers, ammonioalkyl methacrylate copolymers, and polyvinylpyrrolidones.

According to certain embodiments, the total amount of polymer contained in the fingolimod-containing layer ranges from about 50 % to about 99 % by weight, preferably from about 60 % to about 99 % by weight, more preferably from about 70 % to about 99 % by weight based on the fingolimod-containing layer.

When using an additional skin contact layer, the ingredients of the fingolimod-containing layer such as the fingolimod and optional additional active agents, optional auxiliary polymers, optional anti-oxidants, and optional additional excipients or additives may over time migrate into the additional skin contact layer. This however depends on the ingredients and the material of the skin contact layer.

According to particular embodiments, the fingolimod-containing layer does not comprise a fatty acid ester.

According to particular embodiments, the fingolimod-containing layer does not comprise an ester of dodecanol (e.g. lauryl lactate).

According to particular embodiments, the fingolimod-containing layer does not comprise an organosulfur compound (e.g. DMSO).

According to particular embodiments, the fingolimod-containing layer does not comprise an ester of dodecanol, an organosulfur compound, and a fatty acid ester.

In one embodiment of the present invention, the fingolimod-containing layer consists of fingolimod, dodecan-1-ol, and polymer (e.g. polymer-based pressure sensitive adhesive).

## POLYMER

The fingolimod-containing layer according to the present invention comprises at least one polymer. The polymer may be selected from the group consisting of a silicone acrylic hybrid polymer, a polymer based on polysiloxanes, a polymer based on polyisobutylenes, and an acrylate polymer.

In a preferred embodiment, the at least one polymer is a polymer-based pressure-sensitive adhesive.

According to one embodiment of the invention, the at least one polymer is a silicone acrylic hybrid polymer. The silicone acrylic hybrid polymer comprises a polymerized hybrid species that includes silicone-based sub-species and acrylate-based sub-species that have been polymerized together. The silicone acrylic hybrid polymer thus comprises a silicone phase and an acrylic phase. Preferably, the silicone acrylic hybrid polymer is a silicone acrylic hybrid pressure-sensitive adhesive.

The silicone acrylic hybrid pressure-sensitive adhesives are usually supplied and used in solvents like n-heptane and ethyl acetate. The solids content of the pressure-sensitive adhesives is usually between 30 % and 80 %. The skilled person is aware that the solids content may be modified by adding a suitable amount of solvent.

Preferably, the weight ratio of silicone to acrylate in the silicone acrylic hybrid pressure-sensitive adhesive is from 5:95 to 95:5, or from 20:80 to 80:20, more preferably from 40:60 to 60:40, and most preferably the ratio of silicone to acrylate is about 50:50. Suitable silicone acrylic hybrid pressure-sensitive adhesives having a weight ratio of silicone to acrylate of 50:50 are, for example, the commercially available silicone acrylic hybrid pressure-sensitive adhesives 7-6102, Silicone/Acrylate Ratio 50/50, and 7-6302, Silicone/Acrylate Ratio 50/50, supplied in ethyl acetate by Dow Coming.

Suitable silicone acrylic hybrid pressure-sensitive adhesives which are commercially available include the PSA series 7-6100 and 7-6300 manufactured and supplied in n-heptane or ethyl acetate by Dow Corning (7-610X and 7-630X; X=1 n-heptane-based / X=2 ethyl acetate-based). For example, the 7-6102 silicone acrylic hybrid PSA having a silicone/acrylate ratio of 50/50 is characterized by a solution viscosity at 25 °C and about 50 % solids content in ethyl acetate of 2,500 cP and a complex viscosity at 0.1 rad/s at 30 °C of 1.0e7 Poise. The 7-6302 silicone acrylic hybrid PSA having a silicone/acrylate ratio of 50/50 has a solution viscosity at 25 °C and about 50 % solids content in ethyl acetate of 1,500 cP and a complex viscosity at 0.1 rad/s at 30 °C of 4.0e6 Poise.

Depending on the solvent in which the silicone acrylic hybrid pressure-sensitive adhesive is supplied, the arrangement of the silicone phase and the acrylic phase providing a silicone or acrylic continuous external phase and a corresponding discontinuous internal phase is different. If the silicone acrylic hybrid pressure-sensitive adhesive is provided in n-heptane, the composition contains a continuous, silicone external phase and a discontinuous, acrylic internal phase. If the silicone acrylic hybrid pressure-sensitive adhesive is provided in ethyl acetate, the composition contains a continuous, acrylic external phase and a discontinuous, silicone internal phase. After evaporating the solvent in which the silicone acrylic hybrid pressure-sensitive adhesive is provided, the phase arrangement of the resulting pressure-sensitive adhesive film or layer corresponds to the phase arrangement of the solvent-containing adhesive coating composition. For example, in the absence of any substance that may induce an inversion of the phase arrangement in a silicone acrylic hybrid pressure sensitive adhesive composition, a pressure-sensitive adhesive layer prepared from a silicone acrylic hybrid pressure-sensitive adhesive in n-heptane provides a continuous, silicone external phase and a discontinuous, acrylic internal phase, a pressure-sensitive adhesive layer prepared from a silicone acrylic hybrid pressure-sensitive adhesive in ethyl acetate provides a continuous, acrylic external phase and a discontinuous, silicone internal phase. The phase arrangement of the compositions can, for example, be determined in peel force tests with pressure-sensitive adhesive films or layers prepared from the silicone acrylic hybrid PSA compositions which are attached to a siliconized release liner. The pressure-sensitive adhesive film contains a continuous, silicone external phase if the siliconized release liner cannot or can only hardly be removed from the pressure-sensitive adhesive film (laminated to a backing film) due to the blocking of the two silicone surfaces. Blocking results from the adherence of two silicone layers which comprise a similar surface energy. The adhesive shows a good spreading on the siliconized liner and therefore can create a good adhesion to the liner. If the siliconized release liner can easily be removed the pressure-sensitive adhesive film contains a continuous, acrylic external phase. The acrylic adhesive has no good spreading due to the different surface energies and thus has a low or almost no adhesion to the siliconized liner.

According to a preferred embodiment of the invention the silicone acrylic hybrid polymer is a silicone acrylic hybrid pressure-sensitive adhesive obtainable from a silicon-containing pressure-sensitive adhesive composition comprising acrylate or methacrylate functionality. It is to be understood that the silicon-containing pressure-sensitive adhesive composition comprising acrylate or methacrylate functionality can include only acrylate functionality, only methacrylate functionality, or both acrylate functionality and methacrylate functionality.

According to certain embodiments of the invention the silicone acrylic hybrid pressure-sensitive adhesive comprises the reaction product of (a) a silicon-containing pressure-sensitive adhesive composition comprising acrylate or methacrylate functionality, (b) an ethylenically unsaturated monomer, and (c) an initiator. That is, the silicone acrylic hybrid pressure-sensitive adhesive is the product of the chemical reaction between these reactants ((a), (b), and (c)). In particular, the silicone acrylic hybrid pressure-sensitive adhesive includes the reaction product of (a) a silicon-containing pressure-sensitive adhesive composition comprising acrylate or methacrylate functionality, (b) a (meth)acrylate monomer, and (c) an initiator (i.e., in the presence of the initiator). That is, the silicone acrylic hybrid pressure-sensitive adhesive includes the product of the chemical reaction between these reactants ((a), (b), and (c)).

The reaction product of (a) a silicon-containing pressure-sensitive adhesive composition comprising acrylate or methacrylate functionality, (b) an ethylenically unsaturated monomer, and (c) an initiator may contain a continuous, silicone external phase and a discontinuous, acrylic internal phase or the reaction product of (a), (b), and (c) may contain a continuous, acrylic external phase and a discontinuous, silicone internal phase.

The silicon-containing pressure-sensitive adhesive composition comprising acrylate or methacrylate functionality (a) is typically present in the silicone acrylic hybrid pressure-sensitive adhesive in an amount of from 5 to 95, more typically 25 to 75, parts by weight based on 100 parts by weight of the hybrid pressure-sensitive adhesive.

The ethylenically unsaturated monomer (b) is typically present in the silicone acrylic hybrid pressure-sensitive adhesive in an amount of from 5 to 95, more typically 25 to 75, parts by weight based on 100 parts by weight of the hybrid pressure-sensitive adhesive.

The initiator (c) is typically present in the silicone acrylic hybrid pressure-sensitive adhesive in an amount of from 0.005 to 3, more typically from 0.01 to 2, parts by weight based on 100 parts by weight of the hybrid pressure-sensitive adhesive.

According to certain embodiments of the invention the silicon-containing pressure-sensitive adhesive composition comprising acrylate or methacrylate functionality (a) comprises the condensation reaction product of (a1) a silicone resin, (a2) a silicone polymer, and (a3) a silicon-containing capping agent which provides said acrylate or methacrylate functionality. The silicone resin (a1) may also be referred to as silicate resin or silica resin. Preferably, the silicone polymer (a2) is a polysiloxane, preferably polydimethylsiloxane. It is to be understood that (a1) and (a2) form a silicone-based pressure sensitive adhesive by polycondensation, and that the acrylate or methacrylate functionality is introduced by reaction with (a3).

According to certain embodiments of the invention the silicon-containing pressure-sensitive adhesive composition comprising acrylate or methacrylate functionality (a) comprises the condensation reaction product of:

(a1) a silicone resin,
(a2) a silicone polymer, and
(a3) a silicon-containing capping agent which provides said acrylate or methacrylate functionality, wherein said silicon-containing capping agent is of the general formula $XYR'_bSiZ_{3-b}$, wherein

X is a monovalent radical of the general formula AE-
where E is -O- or -NH- and A is an acryl group or a methacryl group,
Y is a divalent alkylene radical having from 1 to 6 carbon atoms,
R' is a methyl or a phenyl radical,
Z is a monovalent hydrolyzable organic radical or a halogen, and
b is 0 or 1;

wherein the silicone resin and silicone polymer are reacted to form a pressure-sensitive adhesive, wherein the silicon-containing capping agent is introduced prior to, during, or after the silicone resin and silicone polymer are reacted, and wherein:

the silicon-containing capping agent reacts with the pressure-sensitive adhesive after the silicone resin and silicone polymer have been condensation reacted to form the pressure-sensitive adhesive; or
the silicon-containing capping agent reacts *in-situ* with the silicone resin and silicone polymer.

According to certain embodiments of the invention the silicon-containing pressure-sensitive adhesive composition comprising acrylate or methacrylate functionality comprises the condensation reaction product of a pressure sensitive adhesive and a silicon-containing capping agent which provides said acrylate or methacrylate functionality. That is, the silicon-containing pressure sensitive adhesive composition comprising acrylate or methacrylate functionality is essentially a pressure sensitive adhesive that has been capped or end blocked with the silicon-containing capping agent which provides said acrylate or methacrylate functionality, wherein the pressure sensitive adhesive comprises the condensation reaction product of the silicone resin and the silicone polymer. Preferably, the silicone resin reacts in an amount of from 30 to 80 parts by weight to form the pressure sensitive adhesive, and the silicone polymer reacts in an amount of from 20 to 70 parts by weight to form the pressure sensitive adhesive. Both of these parts by weight are based on 100 parts by weight of the pressure sensitive adhesive. Although not required, the pressure sensitive adhesive may comprise a catalytic amount of a condensation catalyst. A wide array of silicone resins and silicone polymers are suitable to make up the pressure sensitive adhesive.

According to certain embodiments of the invention the silicone acrylic hybrid pressure-sensitive adhesive is the reaction product of:

(a) a silicon-containing pressure-sensitive adhesive composition comprising acrylate or methacrylate functionality that comprises the condensation reaction product of:

(a1) a silicone resin,
(a2) a silicone polymer, and
(a3) a silicon-containing capping agent which provides said acrylate or methacrylate functionality, wherein said silicon-containing capping agent is of the general formula $XYR'_bSiZ_{3-b}$, wherein

X is a monovalent radical of the general formula AE-
where E is -O- or -NH- and A is an acryl group or a methacryl group,
Y is a divalent alkylene radical having from 1 to 6 carbon atoms,
R' is a methyl or a phenyl radical,
Z is a monovalent hydrolyzable organic radical or a halogen, and
b is 0 or 1;

wherein the silicone resin and silicone polymer are reacted to form a pressure-sensitive adhesive, wherein the silicon-containing capping agent is introduced prior to, during, or after the silicone resin and silicone polymer are reacted, and wherein:

the silicon-containing capping agent reacts with the pressure-sensitive adhesive after the silicone resin and silicone polymer have been condensation reacted to form the pressure-sensitive adhesive; or
the silicon-containing capping agent reacts in-situ with the silicone resin and silicone polymer;

(b) an ethylenically unsaturated monomer; and
(c) an initiator.

The silicone acrylic hybrid composition used in the present invention may be described by being prepared by a method comprising the steps of:

(i) providing a silicon-containing pressure-sensitive adhesive composition comprising acrylate or methacrylate functionality that comprises the condensation reaction product of:

a silicone resin,
a silicone polymer, and
a silicon-containing capping agent which provides said acrylate or methacrylate functionality, wherein said silicon-containing capping agent is of the general formula $XYR'_bSiZ_{3-b}$, wherein

X is a monovalent radical of the general formula AE-
where E is -O- or -NH- and A is an acryl group or a methacryl group,
Y is a divalent alkylene radical having from 1 to 6 carbon atoms,
R' is a methyl or a phenyl radical,
Z is a monovalent hydrolyzable organic radical or a halogen, and
b is 0 or 1;

wherein the silicone resin and silicone polymer are reacted to form a pressure-sensitive adhesive, wherein the silicon-containing capping agent is introduced prior to, during, or after the silicone resin and silicone polymer are reacted, and wherein:

the silicon-containing capping agent reacts with the pressure-sensitive adhesive after the silicone resin and silicone polymer have been condensation reacted to form the pressure-sensitive adhesive; or
the silicon-containing capping agent reacts in-situ with the silicone resin and silicone polymer;

(ii) polymerizing an ethylenically unsaturated monomer and the silicon-containing pressure-sensitive adhesive composition comprising acrylate or methacrylate functionality of step (i) in the presence of an initiator to form a silicone acrylic hybrid composition, optionally at a temperature of from 50 °C to 100 °C, or from 65 °C to 90 °C.

During the polymerization of the ethylenically unsaturated monomer and the silicon-containing pressure-sensitive adhesive composition comprising acrylate or methacrylate functionality, the silicone to acrylic ratio can be controlled and optimized as desired. The silicone to acrylic ratio can be controlled by a wide variety of mechanisms in and during the method. An illustrative example of one such mechanism is the rate controlled addition of the ethylenically unsaturated monomer or monomers to the silicon-containing pressure-sensitive adhesive composition comprising acrylate or methacrylate functionality. In certain applications, it may be desirable to have the silicone-based sub-species, or the overall silicone content, to exceed the acrylate-based sub-species, or the overall acrylic content. In other applications, it may be desirable for the opposite to be true. Independent of the end application, it is generally preferred, as already described above, that the silicon-containing pressure-sensitive adhesive composition comprising acrylate or methacrylate functionality is preferably present in the silicone acrylic hybrid composition in an amount of from about 5 to about 95, more preferably from about 25 to about 75, and still more preferably from about 40 to about 60 parts by weight based on 100 parts by weight of the silicone acrylic hybrid composition.

According to a certain embodiment of the invention, the silicone acrylic hybrid composition used in the present invention may be described by being prepared by a method comprising the steps of:

(i) providing a silicon-containing pressure-sensitive adhesive composition comprising acrylate or methacrylate functionality that comprises the condensation reaction product of:

a silicone resin,
a silicone polymer, and
a silicon-containing capping agent which provides said acrylate or methacrylate functionality, wherein said silicon-containing capping agent is of the general formula $XYR'_bSiZ_{3-b}$, wherein

X is a monovalent radical of the general formula AE-
where E is -O- or -NH- and A is an acryl group or a methacryl group,
Y is a divalent alkylene radical having from 1 to 6 carbon atoms,
R' is a methyl or a phenyl radical,
Z is a monovalent hydrolyzable organic radical or a halogen, and b is 0 or 1;

wherein the silicone resin and silicone polymer are reacted to form a pressure-sensitive adhesive, wherein the silicon-containing capping agent is introduced prior to, during, or after the silicone resin and silicone polymer are reacted, and wherein:

the silicon-containing capping agent reacts with the pressure-sensitive adhesive after the silicone resin and silicone polymer have been condensation reacted to form the pressure-sensitive adhesive; or
the silicon-containing capping agent reacts in-situ with the silicone resin and silicone polymer;

(ii) polymerizing an ethylenically unsaturated monomer and the silicon-containing pressure-sensitive adhesive composition comprising acrylate or methacrylate functionality of step (i) in a first solvent in the presence of an initiator at a temperature of from 50 °C to 100 °C to form a silicone acrylic hybrid composition;

(iii) removing the first solvent; and

(iv) adding a second solvent to form the silicone acrylic hybrid composition, wherein the phase arrangement of the silicone acrylic hybrid composition is selectively controlled by selection of the second solvent.

The silicone acrylic hybrid PSA composition used in the present invention may also be described by being prepared by a method comprising the steps of:

(i) providing a silicon-containing pressure-sensitive adhesive composition comprising acrylate or methacrylate functionality that comprises the condensation reaction product of:

a silicone resin,

a silicone polymer, and

a silicon-containing capping agent which provides said acrylate or methacrylate functionality, wherein said silicon-containing capping agent is of the general formula $XYR'_bSiZ_{3-b}$, wherein

X is a monovalent radical of the general formula AE-

where E is -O- or -NH- and A is an acryl group or a methacryl group,

Y is a divalent alkylene radical having from 1 to 6 carbon atoms,

R' is a methyl or a phenyl radical,

Z is a monovalent hydrolyzable organic radical or a halogen, and b is 0 or 1;

wherein the silicone resin and silicone polymer are reacted to form a pressure-sensitive adhesive, wherein the silicon-containing capping agent is introduced prior to, during, or

after the silicone resin and silicone polymer are reacted, and wherein:

the silicon-containing capping agent reacts with the pressure-sensitive adhesive after the silicone resin and silicone polymer have been condensation reacted to form the pressure-sensitive adhesive; or

the silicon-containing capping agent reacts in-situ with the silicone resin and silicone polymer;

(ii) polymerizing an ethylenically unsaturated monomer and the silicon-containing pressure-sensitive adhesive composition comprising acrylate or methacrylate functionality of step (i) in a first solvent in the presence of an initiator at a temperature of from 50 °C to 100 °C to form a silicone acrylic hybrid composition;

(iii) adding a processing solvent, wherein the processing solvent has a higher boiling point than the first solvent, and

(iv) applying heat at a temperature of from 70 °C to 150 °C such that a majority of the first solvent is selectively removed;

(v) removing the processing solvent; and.

(vi) adding a second solvent to form the silicone acrylic hybrid composition, wherein the phase arrangement of the silicone acrylic hybrid composition is selectively controlled by selection of the second solvent.

The silicone resin according to the previous paragraphs may contain a copolymer comprising triorganosiloxy units of the formula $R^X_3SiO_{1/2}$ and tetrafunctional siloxy units of the formula $SiO_{4/2}$ in a ratio of from 0.1 to 0.9, preferably of about 0.6 to 0.9, triorganosiloxy units for each tetrafunctional siloxy unit. Preferably, each $R^X$ independently denotes a monovalent hydrocarbon radical having from 1 to 6 carbon atoms, vinyl, hydroxyl or phenyl groups.

The silicone polymer according to the previous paragraphs may comprise at least one polydiorganosiloxane and is preferably end-capped (end-blocked) with a functional group selected from the group consisting of hydroxyl groups, alkoxy groups, hydride groups, vinyl groups, or mixtures thereof. The diorganosubstituent may be selected from the group consisting of dimethyl, methylvinyl, methylphenyl, diphenyl, methylethyl, (3,3,3-trifluoropropyl)methyl and mixtures thereof. Preferably, the diorganosubstituents contain only methyl groups. The molecular weight of polydiorganosiloxane will typically range from about 50,000 to about 1,000,000, preferably, from about 80,000 to about 300,000. Preferably, the polydiorganosiloxane comprises $AR^XSiO$ units terminated with endblocking $TR^XASiO_{1/2}$ units, wherein the polydiorganosiloxane has a viscosity of from about 100 centipoise to about 30,000,000 centipoise at 25 °C, each A radical is independently selected from $R^X$ or halohydrocarbon radicals having from 1 to 6 carbon atoms, each T radical is independently selected from the group consisting of $R^X$, OH, H or $OR^Y$, and each $R^Y$ is independently an alkyl radical having from 1 to 4 carbon atoms.

As an example using forms of the preferred silicone resin and the preferred silicone polymer, one type of pressure sensitive adhesive is made by:

mixing (i) from 30 to 80 inclusive parts by weight of at least one resin copolymer containing silicon-bonded hydroxyl radicals and consisting essentially of $R^X_3SiO_{1/2}$ units and $SiO_{4/2}$ units in a mole ratio of 0.6 to 0.9 $R^X_3SiO_{1/2}$ units for each $SiO_{4/2}$ unit present, (ii) between about 20 and

about 70 parts by weight of at least one polydiorganosiloxane comprising $AR^XSiO$ units terminated with endblocking $TR^XASiO_{1/2}$ units, wherein the polydiorganosiloxane has a viscosity of from about 100 centipoise to about 30,000,000 centipoise at 25 °C and each $R^X$ is a monovalent organic radical selected from the group consisting of hydrocarbon radicals of from 1 to 6 inclusive carbon atoms, each A radical is independently selected from $R^X$ or halohydrocarbon radicals having from 1 to 6 inclusive carbon atoms, each T radical is independently selected from the group consisting of $R^X$, OH, H or $OR^Y$, and each $R^Y$ is independently an alkyl radical of from 1 to 4 inclusive carbon atoms; a sufficient amount of (iii) at least one of the silicon-containing capping agents, also referred to throughout as endblocking agents, described below and capable of providing a silanol content, or concentration, in the range of 5,000 to 15,000, more typically 8,000 to 13,000, ppm, when desirable an additional catalytic amount of (iv) a mild silanol condensation catalyst in the event that none is provided by (ii), and

when necessary, an effective amount of (v) an organic solvent which is inert with respect to (i), (ii), (iii) and (iv) to reduce the viscosity of a mixture of (i), (ii), (iii), and (iv), and condensing the mixture of (i), (ii), (iii) and (iv) at least until a substantial amount of the silicon-containing capping agent or agents have reacted with the silicon-bonded hydroxyl radicals and T radicals of (i) and (ii). Additional organosilicon endblocking agents can be used in conjunction with the silicon-containing capping agent or agents (iii) of the present invention.

The silicon-containing capping agent according to the previous paragraphs may be selected from the group of acrylate functional silanes, acrylate functional silazanes, acrylate functional disilazanes, acrylate functional disiloxanes, methacrylate functional silanes, methacrylate functional silazanes, methacrylate functional disilazanes, meth-acrylate functional disiloxanes, and combinations thereof and may be described as to be of the general formula $XYR'_bSiZ_{3-b}$, wherein X is a monovalent radical of the general formula AE- where E is -O- or -NH- and A is an acryl group or a methacryl group, Y is a divalent alkylene radical having from 1 to 6 carbon atoms, R' is a methyl or a phenyl radical, Z is a monovalent hydrolyzable organic radical or a halogen, and b is 0, 1 or 2. Preferably, the monovalent hydrolyzable organic radical is of the general formula R"0 - where R" is an alkylene radical. Most preferably, this particular endblocking agent is selected from the group of 3-methacryloxypropyldimethylchlorosilane, 3- methacryloxypropyldichlorosilane, 3-methacryloxypropyltrichlorosilane, 3-methacryloxypropyldimethylmethoxysilane, 3-methacryloxypropylmethyldimethoxysilane, 3-methacryloxypropyltrimethoxysilane, 3-methacryloxypropyldimethylethoxysilane, 3-methacryloxypropylmethyldiethoxysilane, 3-methacryloxypropyltriethoxysilane, (methacryloxymethyl)dimethylmethoxysilane, (methacryloxymethyl)methyldimethoxysilane, (methacryloxymethyl)trimethoxysilane, (methacryloxymethyl)dimethylethoxysilane, (methacryloxymethyl)methyldiethoxysilane, methacryloxymethyltriethoxysilane, methacryloxypropyltriisopropoxysilane, 3-methacryloxypropyldimethylsilazane, 3-acryloxypropyldimethylchlorosilane, 3-acryloxypropyldichlorosilane, 3-acryloxypropyl-trichlorosilane, 3-acryloxypropyldimethylmethoxysilane, 3-acryloxy-propylmethyldimethoxysilane, 3-acryloxypropyltrimethoxysilane, 3-acryloxypropyl-dimethylsilazane, and combinations thereof.

The ethylenically unsaturated monomer according to the previous paragraphs can be any monomer having at least one carbon-carbon double bond. Preferably, the ethylenically unsaturated monomer according to the previous paragraphs may be a compound selected from the group consisting of aliphatic acrylates, aliphatic methacrylates, cycloaliphatic acrylates, cycloaliphatic methacrylates, and combinations thereof. It is to be understood that each of the compounds, the aliphatic acrylates, the aliphatic methacrylates, the cycloaliphatic acrylates, and the cycloaliphatic methacrylates, include an alkyl radical. The alkyl radicals of these compounds can include up to 20 carbon atoms. The aliphatic acrylates that may be selected as one of the ethylenically unsaturated monomers are selected from the group consisting of methyl acrylate, ethyl acrylate, propyl acrylate, n-butyl acrylate, iso-butyl acrylate, tert-butyl acrylate, hexyl acrylate, 2-ethylhexyl acrylate, iso-octyl acrylate, iso-nonyl acrylate, iso-pentyl acrylate, tridecyl acrylate, stearyl acrylate, lauryl acrylate, and mixtures thereof. The aliphatic methacrylates that may be selected as one of the ethylenically unsaturated monomers are selected from the group consisting of methyl methacrylate, ethyl methacrylate, propyl methacrylate, n-butyl methacrylate, iso-butyl meth-acrylate, tert-butyl methacrylate, hexyl methacrylate, 2-ethylhexyl methacrylate, iso-octyl methacrylate, iso-nonyl methacrylate, iso-pentyl methacrylate, tridecyl methacrylate, stearyl methacrylate, lauryl methacrylate, and mixtures thereof. The cycloaliphatic acrylate that may be selected as one of the ethylenically unsaturated monomers is cyclohexyl acrylate, and the cycloaliphatic methacrylate that may be selected as one of the ethylenically unsaturated monomers is cyclohexyl methacrylate.

It is to be understood that the ethylenically unsaturated monomer used for preparing the silicone acrylic hybrid pressure sensitive adhesive may be more than one ethylenically unsaturated monomer. That is, a combination of ethylenically unsaturated monomers may be polymerized, more specifically co-polymerized, along with the silicon-containing pressure sensitive adhesive composition comprising acrylate or methacrylate functionality and the initiator. According to a certain embodiment of the invention, the silicone acrylic hybrid pressure-sensitive adhesive is prepared by using at least two different ethylenically unsaturated monomers, preferably selected from the group of 2-ethylhexyl acrylate and methyl acrylate, preferably in a ratio of from 40:60 to 70:30, more preferably in a ratio of from 65:35 to 55:45 or of from 55:45 to 45:50, particular preferred in a ratio of 50 % 2-ethylhexyl acrylate and 50 % methyl acrylate, or in a ratio of 60 % 2-ethylhexyl acrylate and 40 % methyl acrylate, as the acrylic monomer.

The initiator according to the previous paragraphs may be any substance that is suitable to initiate the polymerization of the silicon-containing pressure sensitive adhesive composition comprising acrylate or methacrylate functionality and the ethylenically unsaturated monomer to form the silicone acrylic hybrid. For example, free radical initiators selected from the group of peroxides, azo compounds, redox initiators, and photo-initiators may be used.

Further suitable silicone resins, silicone polymers, silicon-containing capping agents, ethylenically unsaturated monomers, and initiators that can be used in accordance with the previous paragraphs are detailed in WO 2007/145996, EP 2 599 847 A1, and WO 2016/130408.

According to a certain embodiment of the invention, the silicone acrylic hybrid polymer comprises a reaction product of a silicone polymer, a silicone resin and an acrylic polymer, wherein the acrylic polymer is covalently self-crosslinked and covalently bound to the silicone polymer and/or the silicone resin.

According to a certain other embodiment of the invention, the silicone acrylic hybrid polymer comprises a reaction product of a silicone polymer, a silicone resin and an acrylic polymer, wherein the silicone resin contains triorganosiloxy units $R_3SiO_{1/2}$ where R is an organic group, and tetrafunctional siloxy units $SiO_{4/2}$ in a mole ratio of from 0.1 to 0.9 $R_3SiO_{1/2}$ units for each $SiO_{4/2}$.

The acrylic polymer may comprise at least an alkoxysilyl functional monomer, polysiloxane-containing monomer, halosilyl functional monomer or alkoxy halosilyl functional monomer. Preferably, the acrylic polymer is prepared from alkoxysilyl functional monomers selected from the group consisting of trialkoxylsilyl (meth)acrylates, dialkoxyalkylsilyl (meth)acrylates, and mixtures thereof, or comprises end-capped alkoxysilyl functional groups. The alkoxysilyl functional groups may preferably be selected from the group consisting of trimethoxylsilyl groups, dimethoxymethylsilyl groups, triethoxylsilyl, diethoxymethylsilyl groups and mixtures thereof.

The acrylic polymer may also be prepared from a mixture comprising polysiloxane-containing monomers, preferably from a mixture comprising polydimethylsiloxane mono (meth)acrylate.

The silyl functional monomers will typically be used in amounts of from 0.2 to 20 % by weight of the acrylic polymer, more preferably the amount of silyl functional monomers will range from about 1.5 to about 5 % by weight of the acrylic polymer.

The amount of polysiloxane-containing monomer will typically be used in amounts of from 1.5 to 50 % by weight of the acrylic polymer, more preferably the amount of polysiloxane-containing monomers will range from 5 to 15 % by weight of the acrylic polymer.

Alternatively, the acrylic polymer comprises a block or grafted copolymer of acrylic and polysiloxane. An example of a polysiloxane block copolymer is polydimethylsiloxaneacrylic block copolymer. The preferred amount of siloxane block is 10 to 50 % by weight of the whole block polymer.

The acrylic polymer comprises alkyl (meth)acrylate monomers. Preferred alkyl (meth)acrylates which may be used have up to about 18 carbon atoms in the alkyl group, preferably from 1 to about 12 carbon atoms in the alkyl group. Preferred low glass transition temperature (Tg) alkyl acrylate with a homopolymer Tg of less than about 0 °C have from about 4 to about 10 carbon atoms in the alkyl group and include butyl acrylate, amyl acrylate, hexyl acrylate, 2-ethylhexyl acrylate, octyl acrylate, isooctyl acrylate, decyl acrylate, isomers thereof, and combinations thereof. Particularly preferred are butyl acrylate, 2-ethylhexyl acrylate and isooctyl acrylate. The acrylic polymer components may further comprise (meth)acrylate monomers having a high Tg such as methyl acrylate, ethyl acrylate, methyl methacrylate and isobutyl methacrylate.

The acrylic polymer component may further comprise a polyisobutylene group to improve cold flow properties of the resultant adhesive.

The acrylic polymer components may comprise nitrogen-containing polar monomers. Examples include N-vinyl pyrrolidone, N-vinyl caprolactam, N-tertiary octyl acrylamide, dimethyl acrylamide, diacetone acrylamide, N-tertiary butyl acrylamide, N-isopropyl acrylamide, cyanoethylacrylate, N-vinyl acetamide and N-vinyl formamide.

The acrylic polymer component may comprise one or more hydroxyl containing monomers such as 2-hydroxyethyl acrylate, 2-hydroxyethyl methacrylate, hydroxypropyl acrylate and/or hydroxypropyl methacrylate.

The acrylic polymer components may, if desired, comprise carboxylic acid containing monomers. Useful carboxylic acids preferably contain from about 3 to about 6 carbon atoms and include, among others, acrylic acid, methacrylic acid, itaconic acid, β-carboxyethyl acrylate and the like. Acrylic acid is particularly preferred.

Other useful, well known co-monomers include vinyl acetate, styrene, cyclohexyl acrylate, alkyl di(meth)acrylates, glycidyl methacrylate and allyl glycidyl ether, as well as macromers such as, for example, poly(styryl)methacrylate.

One acrylic polymer component that can be used in the practice of the invention is an acrylic polymer that comprises from about 90 to about 99.5 % by weight of butyl acrylate and from about 0.5 to about 10 % by weight dimethoxymethylsilyl methacrylate.

According to a certain embodiment of the invention the silicone acrylic hybrid polymer may be prepared by a) reacting silicone polymer with silicone resin to form a resultant product, b) reacting the resultant product of a) with an acrylic polymer containing reactive functionality, wherein the components are reacted in an organic solvent.

According to a certain embodiment of the invention the silicone acrylic hybrid polymer may be prepared by a) reacting a silicone resin with an acrylic polymer containing reactive functionality to form a resultant product, b) reacting the resultant product of a) with silicone polymer, wherein the components are reacted in an organic solvent.

According to a certain embodiment of the invention the silicone acrylic hybrid polymer may be prepared by a) reacting a silicone polymer with an acrylic polymer containing reactive functionality to form a resultant product, b) reacting the resultant product of a) with silicone resin, wherein the components are reacted in an organic solvent.

Further suitable acrylic polymers, silicone resins, and silicone polymers that can be used for chemically reacting together a silicone polymer, a silicone resin and an acrylic polymer to provide a silicone acrylic hybrid polymer in accordance with the previous paragraphs are detailed in WO 2010/124187.

According to certain embodiments of the invention, the at least one polymer is a non-hybrid polymer. Non-hybrid polymers (e.g. non-hybrid pressure-sensitive adhesives) are polymers (e.g. polymer-based pressure-sensitive adhesives) which do not include a hybrid species. Preferred are non-hybrid polymers (e.g. non-hybrid pressure-sensitive adhesives) based on polysiloxanes, polyisobutylenes, and acrylates.

The non-hybrid polymers (e.g. the non-hybrid pressure-sensitive adhesives) may be contained in the active agent-containing layer structure and/or in the adhesive overlay.

Non-hybrid pressure-sensitive adhesives are usually supplied and used in solvents like n-heptane and ethyl acetate. The solids content of the pressure-sensitive adhesives is usually between 30 % and 80 %.

Suitable non-hybrid polymers according to the invention are commercially available e.g. under the brand names BIO-PSAs (pressure-sensitive adhesives based on polysiloxanes), Oppanol™ (polyisobutylenes), or Duro-Tak™ (acrylic polymers).

According to one embodiment of the invention, the at least one polymer is a polymer based on polysiloxanes. Polymers based on polysiloxanes may also be referred to as silicone-based polymers or polysiloxane-based polymers. Suitable polymers based on polysiloxanes are preferably pressure sensitive adhesives based on polysiloxanes. Pressure-sensitive adhesives based on polysiloxanes may also be referred to as silicone-based adhesives, silicone-based pressure-sensitive adhesives, polysiloxane-based adhesives, or polysiloxane-based pressure-sensitive adhesives. These pressure-sensitive adhesives based on polysiloxanes provide for suitable tack and for quick bonding to various skin types, including wet skin, suitable adhesive and cohesive qualities, long lasting adhesion to the skin, a high degree of flexibility, a permeability to moisture, and compatibility to many actives and film-substrates. It is possible to provide them with sufficient amine resistance and therefore enhanced stability in the presence of amines. Such pressure-sensitive adhesives are based on a resin-in-polymer concept wherein, by condensation reaction of silanol end blocked polydimethylsiloxane with a silica resin (also referred to as silicate resin), a pressure-sensitive adhesive based on polysiloxane is prepared wherein for amine stability the residual silanol functionality is additionally capped with trimethylsiloxy groups. The silanol end blocked polydimethylsiloxane content contributes to the viscous component of the visco-elastic behavior, and impacts the wetting and the spreadability properties of the adhesive. The resin acts as a tackifying and reinforcing agent, and participates in the elastic component. The correct balance between silanol end blocked polydimethylsiloxane and resin provides for the correct adhesive properties.

Examples of silicone-based PSA compositions which are commercially available include the standard BIO-PSA series (7-4400, 7-4500 and 7-4600 series) and the amine compatible (endcapped) BIO-PSA series (7-4100, 7-4200 and 7-4300 series) manufactured and typically supplied in n-heptane or ethyl acetate by Dow Coming. For example, BIO-PSA 7-4201 is characterized by a solution viscosity at 25 °C and about 60 % solids content in heptane of 450 mPa s and a complex viscosity at 0.01 rad/s at 30 °C of $1 \times 10^8$ Poise. BIO-PSA 7-4301 has a solution viscosity at 25 °C and about 60 % solids content in heptane of 500 mPa s and a complex viscosity at 0.01 rad/s at 30 °C of $5 \times 10^6$ Poise.

The pressure-sensitive adhesives based on polysiloxanes are supplied and used in solvents like n-heptane, ethyl acetate or other volatile silicone fluids. The solids content of pressure-sensitive adhesives based on polysiloxanes in solvents is usually between 60 and 85 %, preferably between 70 and 80 % or between 60 and 75 %. The skilled person is aware that the solids content may be modified by adding a suitable amount of solvent.

According to one embodiment of the invention, the at least one polymer is a polymer based on polyisobutylenes, preferably a pressure-sensitive adhesive based on polyisobutylenes. Suitable polyisobutylenes according to the invention are available under the tradename Oppanol®. Combinations of high molecular weight polyisobutylenes (e.g. N100, N80) and medium molecular weight polyisobutylenes (e.g. B10, B12) may be used. Suitable ratios of medium molecular weight polyisobutylene to high molecular weight polyisobutylene are in the range of from 100:1 to 1:100, preferably from 95:5 to 40:60, more preferably from 90:10 to 80:20. A preferred example for a polyisobutylene combination is B10:N100 in a ratio of 85:15. Oppanol® N100 has a viscosity average molecular weight $M_v$ of 1,110,000, and a weight average molecular weight $M_w$ of 1,550,000, and an average molecular weight distribution $M_w/M_n$ of 2.9. Oppanol® B10 has a viscosity average molecular weight $M_v$ of 40,000, and a weight average molecular weight $M_w$ of 53,000, and an average molecular weight distribution $M_w/M_n$ of 3.2. In certain embodiments, polybutene may be added to the polyisobutylenes. The solids content of polyisobutylenes in solvents is usually between 30 and 50 %, preferably between 35 and 40 %.

The skilled person is aware that the solids content may be modified by adding a suitable amount of solvent.

According to one embodiment of the invention, the at least one polymer is a polymer based on acrylates, preferably a pressure-sensitive adhesive based on acrylates. Pressure-sensitive adhesives based on acrylates may also be referred to as acrylate-based pressure-sensitive adhesives, or acrylate pressure-sensitive adhesives. Pressure-sensitive adhesives based on acrylates may have a solids content preferably between 30 % and 60 %. Such acrylate-based pressure-sensitive adhesives may or may not comprise functional groups such as hydroxy groups, carboxylic acid groups, neutralized carboxylic acid groups and mixtures thereof. Thus, the term "functional groups" in particular refers to hydroxy- and carboxylic acid groups, and deprotonated carboxylic acid groups.

Corresponding commercial products are available e.g. from Henkel under the tradename Duro Tak®. Such acrylate-based pressure-sensitive adhesives are based on monomers selected from one or more of acrylic acid, butylacrylate, 2-ethylhexylacrylate, glycidylmethacrylate, 2-hydroxyethylacrylate, methylacrylate, methylmethacrylate, t-octylacrylamide and vinylacetate, and are provided in ethyl acetate, heptanes, n-heptane, hexane, methanol, ethanol, isopropanol, 2,4-pentanedione, toluene or xylene or mixtures thereof. Suitable acrylate-based pressure-sensitive adhesives are based on monomers selected from two or more of acrylic acid, butylacrylate, 2-ethylhexylacrylate, glycidylmethacrylate, 2-hydroxyethylacrylate, methylacrylate, methylmethacrylate, t-octylacrylamide and vinylacetate.

In a preferred embodiment, the acrylate-based pressure-sensitive adhesive does not contain vinylacetate.

In one embodiment of the present invention, the at least one polymer is an acrylate-based pressure-sensitive adhesive, which is a copolymer based on 2-ethylhexyl-acrylate, 2-hydroxyethyl-acrylate and methylacrylate.

Specific acrylate-based pressure-sensitive adhesives are available as:

- Duro-Tak™ 387-2510 or Duro-Tak™ 87-2510 (a copolymer based on 2-ethylhexyl-acrylate, 2-hydroxyethyl-acrylate and methylacrylate, provided as a solution in ethyl acetate and hexane),
- Duro-Tak™ 87-4287 (a copolymer based on vinyl acetate, 2-ethylhexyl-acrylate, and 2-hydroxyethyl-acrylate provided as a solution in ethyl acetate without cross-linking agent),
- Duro-Tak™ 387-2287 or Duro-Tak™ 87-2287 (a copolymer based on vinyl acetate, 2-ethylhexyl-acrylate, 2-hydroxyethyl-acrylate and glycidyl-methacrylate provided as a solution in ethyl acetate without cross-linking agent),
- Duro-Tak™ 387-2516 or Duro-Tak™ 87-2516 (a copolymer based on vinyl acetate, 2-ethylhexyl-acrylate, 2-hydroxyethyl-acrylate and glycidyl-methacrylate provided as a solution in ethyl acetate, ethanol, n-heptane and methanol with a titanium cross-linking agent),
- Duro-Tak™ 387-2051 or Duro-Tak™ 87-2051 (a copolymer based on acrylic acid, butylacrylate, 2-ethylhexylacrylate and vinyl acetate, provided as a solution in ethyl acetate and heptane),
- Duro-Tak™ 387-2353 or Duro-Tak™ 87-2353 (a copolymer based on acrylic acid, 2-ethylhexylacrylate, glycidylmethacrylate and methylacrylate, provided as a solution in ethyl acetate and hexane),
- Duro-Tak™ 87-4098 (a copolymer based on 2-ethylhexyl-acrylate and vinyl acetate, provided as a solution in ethyl acetate).

In a preferred embodiment, the acrylate-based pressure-sensitive adhesive does not contain a cross-linking agent.

In a preferred embodiment of the present invention, the acrylate-based pressure-sensitive adhesive does not contain carboxyl groups as functional groups.

In one embodiment, the acrylate-based pressure-sensitive adhesive is free of functional groups.

Auxiliary polymers may be added, for example, to enhance cohesion and/or adhesion, or to reduce the cold flow of the polymer layer, as a solubilizer, or as a crystallization inhibitor.

The auxiliary polymer may be selected from the group consisting of alkyl methacrylate copolymers, amino alkyl methacrylate copolymers, methacrylic acid copolymers, methacrylic ester copolymers, ammonioalkyl methacrylate copolymers, polyvinylpyrrolidones, vinylpyrrolidone-vinyl acetate copolymers, polyvinyl caprolactam-polyvinyl acetate-polyethylene glycol copolymer, cellulose derivatives, and mixtures thereof. In one embodiment, the auxiliary polymer is a cellulose derivative, preferably ethyl cellulose.

## RELEASE CHARACTERISTICS

The TTS in accordance with the invention are designed for transdermally administering fingolimod to the systemic circulation for a predefined extended period of time (e.g. at least or about 72 hours, about 84 hours, or about 96 hours, preferably for about 168 hours).

In one aspect, the TTS according to the invention as described above provides a mean release rate of fingolimod of 0.1 to 1.0 mg/day, preferably of 0.3 to 0.7 mg/day, more preferably of 0.4 to 0.6 mg/day over an extended period of time (e.g. at least or about 72 hours, about 84 hours, about 96 hours, or about 168 hours). For example, the TTS according to the invention as described above provides a mean release rate of fingolimod of 0.1 to 1.0 mg/day over at least 72 hours.

In certain embodiments, the TTS according to the invention as described above provides a cumulative permeated amount of fingolimod of more than 1.5 $\mu$g/cm$^2$, or of more than 1.5 $\mu$g/cm$^2$ to 15.0 $\mu$g/cm$^2$, of about 2.0 $\mu$g/cm$^2$ to 15.0 $\mu$g/cm$^2$, 2.5 to 15.0 $\mu$g/cm$^2$, 3.0 to 15.0 $\mu$g/cm$^2$, or 5.0 to 15.0 $\mu$g/cm$^2$ within the first 24 hours of administration as measured in a Franz diffusion cell with dermatomed human skin, preferably determined by measuring the permeated amount of fingolimod in a Franz diffusion cell with dermatomed human skin with a thickness of 500 $\mu$m, when a phosphate buffer solution pH 5.5 with 0.1 % Methyl-$\beta$-Cyclodextrine and 0.1 % saline azide as antibacteriological agent is used at a temperature of 32 $\pm$ 1 °C.

In certain embodiments, the TTS according to the invention as described above provides a cumulative permeated amount of fingolimod of more than 6.0 $\mu$g/cm$^2$, more than 6.0 $\mu$g/cm$^2$ to 40.0 $\mu$g/cm$^2$, of about 7.0 $\mu$g/cm$^2$ to 40.0 $\mu$g/cm$^2$, 9.0 to 40.0 $\mu$g/cm$^2$, 10.0 to 40.0 $\mu$g/cm$^2$, or 15.0 to 40 $\mu$g/cm$^2$ within the first 36 hours of administration as measured in a Franz diffusion cell with dermatomed human skin, preferably determined by measuring the permeated amount of fingolimod in a Franz diffusion cell with dermatomed human skin with a thickness of 500 $\mu$m, when a phosphate buffer solution pH 5.5 with 0.1 % Methyl-$\beta$-Cyclodextrine and 0.1 % saline azide as antibacteriological agent is used at a temperature of 32 $\pm$ 1 °C.

In certain embodiments, the TTS according to the invention as described above provides a skin permeation rate of fingolimod of more than 0.1 $\mu$g/cm$^2$-hr, or of more than 0.1 $\mu$g/cm$^2$-hr to 1.0 $\mu$g/cm$^2$-hr, of about 0.15 $\mu$g/cm$^2$-hr to 1.0 $\mu$g/cm$^2$-hr, 0.2 $\mu$g/cm$^2$-hr to 1.0 $\mu$g/cm$^2$-hr, or 0.2 $\mu$g/cm$^2$-hr to 1.0 $\mu$g/cm$^2$-hr at hour 16 after administration as measured in a Franz diffusion cell with dermatomed human skin, preferably determined by measuring the permeated amount of fingolimod in a Franz diffusion cell with dermatomed human skin with a thickness of 500 $\mu$m, when a phosphate buffer solution pH 5.5 with 0.1 % Methyl-$\beta$-Cyclodextrine and 0.1 % saline azide as antibacteriological agent is used at a temperature of 32 $\pm$ 1 °C.

According to certain aspects of the present invention, the TTS provides a ratio of $C_{max}$ fingolimod phosphate : $C_{max}$ fingolimod of 0.2 : 1 to 0.8 : 1, of 0.3 : 1 to 0.7 : 1, or of about 0.5 : 1 over about 168 hours of administration after a single-dose administration to a subject population.

## METHOD OF TREATMENT/ MEDICAL USE

In accordance with a specific aspect of the present invention, the TTS according to the invention is for use in a method of treating an immune disorder, such as multiple sclerosis.

According to certain aspects of the present invention, the TTS is for use in a method of treating an immune disorder, wherein the transdermal therapeutic system is applied on the skin of a patient for at least or about 72 hours (3 days), or for about 84 hours (3.5 days), or for about 168 hours (7 days). Preferably, the TTS is applied for about 168 hours (7 days).

According to certain aspects of the present invention, the TTS provides a ratio of $C_{max}$ fingolimod phosphate : $C_{max}$ fingolimod of 0.2 : 1 to 0.8 : 1, of 0.3 : 1 to 0.7 : 1, or of about 0.5 : 1 over about 168 hours of administration after a single-dose administration to a subject population.

According to one aspect, the invention relates to fingolimod base for use in a method of treating an immune disorder, preferably multiple sclerosis, wherein fingolimod base is administered to the skin of a patient in a transdermal therapeutic system according to the invention.

According to one aspect, the invention relates to the use of a TTS according to the present invention for the manufacture of a medicament for treating an immune disorder. In particular, the invention relates to the use of a TTS according to the present invention for the manufacture of a medicament for treating multiple sclerosis, wherein preferably the TTS is applied to the skin of a patient for at least of about 72 hours (3 days), or for about 84 hours (3.5 days), or for about 168 hours (7 days).

According to another aspect, the present invention relates to a TTS according to the present invention for use in the treatment of an immune disorder, preferably multiple sclerosis, by applying to the skin of a patient a transdermal therapeutic system according to the invention. In this connection, the TTS as described above is preferably applied to the skin of a patient for at least about 72 hours (3 days), or for about 84 hours (3.5 days), or for about 168 hours (7 days).

## METHOD OF MANUFACTURE

The invention further relates to a method of manufacture of a transdermal therapeutic system according to the invention comprising the steps of:

    1) providing a fingolimod-containing coating composition comprising

        a) fingolimod (e.g. fingolimod base,

b) at least one polymer (e.g. a polymer-based pressure-sensitive adhesive),
c) dodecan-1-ol, and
d) optionally a solvent,

2) coating the fingolimod-containing coating composition onto a release liner in an amount to provide the desired area weight,
3) drying the coated fingolimod-containing coating composition to provide the fingolimod-containing layer,
4) laminating the fingolimod-containing layer to a backing layer to provide a fingolimod-containing layer structure,
5) optionally providing an additional skin contact layer by coating and drying an active agent-free coating composition or an active agent-containing coating composition according to steps 2 and 3, removing the release liner of the fingolimod-containing layer and laminating the adhesive side of the skin contact layer onto the adhesive side of the fingolimod-containing layer to provide a fingolimod-containing layer structure,
6) punching the individual systems from the fingolimod-containing layer structure,
7) optionally adhering to the individual systems an active-free self-adhesive layer structure comprising also a backing layer and an active agent-free pressure-sensitive adhesive layer and which is larger than the individual systems of fingolimod-containing self-adhesive layer structure.

In a preferred embodiment, the at least one polymer is a pressure-sensitive adhesive polymer and is provided as a solution, preferably in ethyl acetate, n-heptane or hexane.

In one embodiment, in step 1) fingolimod is present in the form of fingolimod base and is combined with dodecanol and the at least one polymer in ethyl acetate, n-heptane, or hexane to provide the fingolimod-containing coating composition.

In step 3) and optionally in step 5) of the above method of manufacture, drying is performed preferably at a temperature of from 20 to 90 °C.

## EXAMPLES

The present invention will now be more fully described with reference to the accompanying examples. It should be understood, however, that the following description is illustrative only and should not be taken in any way as a restriction of the invention.

## EXAMPLE 1

### Coating composition

The formulations of the fingolimod base-containing coating compositions are summarized below.

Table 1

| Ingredient (Trade Name) | Comparative Example A | | Comparative Example F | | Example 1 | |
|---|---|---|---|---|---|---|
| | [g] | [%] | [g] | [%] | [g] | [%] |
| Fingolimod base | 0.15 | 3.0 | 0.15 | 3.0 | 0.15 | 3.0 |
| Ethylcellulose | 0.05 | 1.0 | 0.05 | 1.0 | 0.05 | 1.0 |
| Octyldodecanol (Eutanol™ G) | - | - | 0.51 | 10.0 | - | - |
| 1-Dodecanol | - | - | - | - | 0.51 | 10.0 |
| Pressure-sensitive adhesive based on polyisobutylenes in n-heptane; Solids content of 38 % by weight (DURO-TAK™ 87-6908) | 12.52 | 96.0 | 11.27 | 86.0 | 11.24 | 86.0 |
| n-heptane | 0.85 | - | 1.56 | - | 1.55 | - |
| Total | 13.57 | 100.0 | 13.54 | 100.0 | 13.50 | 100.0 |
| Area Weight [g/m²] | 100.1 | | 97.5 | | 97.0 | |

(continued)

| Ingredient (Trade Name) | Comparative Example A | | Comparative Example F | | Example 1 | |
|---|---|---|---|---|---|---|
| | [g] | [%] | [g] | [%] | [g] | [%] |
| Loading API [$\mu$g/cm$^2$] | 302.1 | | 293.5 | | 294.0 | |

**Preparation of the coating composition**

Fingolimod base was put in a suitable mixing vessel (beaker) and n-heptane was added. Subsequently, the pressure-sensitive adhesive (based on polyisobutylenes in the form of a mixture in n-heptane, having a solid content of 38 % by weight, DURO-TAK 87-6908 purchased from Henkel) was added to the slurry. Ethylcellulose (purchased from IMCD) was added to the mixture while stirring and the mass was stirred for approx. 3 hours.

For Comparative Example F, Octyldodecanol (Eutanol™ G purchased from Sigma Aldrich) was added before n-heptane was added.

For Example 1, 1-Dodecanol (purchased from Alfa Aesar) was added before n-heptane was added.

**Coating of the coating composition**

The fingolimod base-containing coating composition was coated within 24 hours on a abhesively equipped polyethylenterephthalate (PET) foil which may function as a release liner and dried at room temperature and 60 °C for 15 minutes each.

The coating thickness was chosen such that removal of the solvents results in an area weight of the matrix layer of approx. 100 g/m$^2$. The dried film was then laminated with a backing layer (polyethylenterephthalate (PET) foil 19 $\mu$m) to provide the fingolimod-containing self-adhesive layer structure.

**Preparation of the TTS (all examples)**

The individual systems (TTS) were then punched out from the fingolimod-containing self-adhesive layer structure.

In specific embodiments a TTS as described above can be provided with an adhesive overlay, i.e. a further self-adhesive layer structure of larger surface area, preferably with rounded corners, comprising a pressure-sensitive adhesive matrix layer which is free of active ingredient and a preferably skin-colored backing layer. The TTSs are then punched out and sealed into pouches of the primary packaging material.

**EXAMPLE 2**

**Coating composition**

The formulations of the fingolimod base-containing coating compositions are summarized below.

Table 2

| Ingredient (Trade Name) | Comparative Example B | | Example 2 | |
|---|---|---|---|---|
| | [g] | [%] | [g] | [%] |
| Fingolimod base | 0.15 | 3.0 | 0.15 | 3.0 |
| Povidone K90F (Kollidon™ 90 F from BASF) | 0.25 | 5.0 | 0.25 | 5.0 |
| 1-Dodecanol | - | - | 0.51 | 10.0 |
| Pressure-sensitive adhesive based on polyisobutylenes in n-heptane; Solids content of 38 % by weight (DURO-TAK™ 87-6908 from Henkel) | 12.07 | 92.0 | 10.72 | 82.0 |
| n-heptane | 1.09 | - | 1.87 | - |
| Total | 13.56 | 100.0 | 13.50 | 100.0 |

(continued)

| Ingredient (Trade Name) | Comparative Example B | | Example 2 | |
|---|---|---|---|---|
| | [g] | [%] | [g] | [%] |
| Area Weight [g/m$^2$] | 101.8 | | 97.8 | |
| Loading API [μg/cm$^2$] | 304.5 | | 298.3 | |

**Preparation of the coating composition**

Fingolimod base was put in a suitable mixing vessel (beaker) and n-heptane was added. The pressure-sensitive adhesive (based on polyisobutylenes in the form of a mixture in n-heptane, having a solid content of 38 % by weight, DURO-TAK 87-6908 purchased from Henkel) was added to the slurry. Polyvinylpyrrolidone (Kollidon™ 90 F from BASF) was added to the mixture while stirring and the mass was stirred for approx. 3 hours.
For Example 2, 1-Dodecanol (purchased from Alfa Aesar) was added before n-heptane was added.

**Coating of the coating composition**

See Example 1.

**Preparation of the TTS**

See Example 1.

**EXAMPLE 3**

**Coating composition**

The formulation of the fingolimod base-containing coating composition is summarized below.

Table 3

| Ingredient (Trade Name) | Comparative Example C | | Example 3 | |
|---|---|---|---|---|
| | [g] | [%] | [g] | [%] |
| Fingolimod base | 0.15 | 3.0 | 0.15 | 3.0 |
| Crospovidone | 1.01 | 20.2 | 1.01 | 20.2 |
| 1-Dodecanol | - | - | 0.52 | 10.2 |
| Pressure-sensitive adhesive based on polyisobutylenes in n-heptane; Solids content of 38 % by weight (DURO-TAK™ 87-6908 from Henkel) | 10.05 | 76.8 | 8.72 | 66.7 |
| n-heptane | 2.29 | - | 3.10 | - |
| Total | 13.50 | 100.0 | 13.50 | 100.0 |
| Area Weight [g/m$^2$] | 104.1 | | 103.2 | |
| Loading API [μg/cm$^2$] | 314.1 | | 308.4 | |

**Preparation of the coating composition**

Fingolimod base was put in a suitable mixing vessel (beaker) and n-heptane was added. The pressure-sensitive adhesive (based on polyisobutylenes in the form of a mixture in n-heptane, having a solid content of 38 % by weight, DURO-TAK 87-6908 purchased from Henkel) was added to the slurry. Crospovidone was added to the mixture while stirring and the mass was stirred for approx. 3 hours.
For Example 3, 1-Dodecanol (purchased from Alfa Aesar) was added before n-heptane was added.

**Coating of the coating composition**

See Example 1.

**Preparation of the TTS**

See Example 1.

**EXAMPLE 4**

**Coating composition**

The formulation of the fingolimod base-containing coating composition is summarized below.

Table 4

| Ingredient (Trade Name) | Comparative Example D | | Example 4 | |
|---|---|---|---|---|
| | [g] | [%] | [g] | [%] |
| Fingolimod base | 0.15 | 3.0 | 0.15 | 3.0 |
| 1-Dodecanol | - | - | 0.50 | 10.0 |
| Acrylic adhesive in ethyl acetate and hexane; Solids content of 41.2 % by weight (DURO-TAK™ 387-2510 from Henkel) | 11.77 | 97.0 | 10.55 | 87.0 |
| n-heptane | 0.62 | - | 1.29 | - |
| Total | 12.54 | 100.0 | 12.49 | 100.0 |
| Area Weight [g/m$^2$] | 111.7 | | 107.1 | |
| Loading API [μg/cm$^2$] | 333.8 | | 320.2 | |

**Preparation of the coating composition**

Fingolimod base was put in a suitable mixing vessel (beaker) and n-heptane was added. The pressure-sensitive adhesive (acrylic adhesive in ethyl acetate and hexane, having a solid content of 41.2 % by weight, DURO-TAK 387-2510 purchased from Henkel) was added to the slurry. The mass was stirred for approx. 3 hours.
For Example 4, 1-Dodecanol (purchased from Alfa Aesar) was added before solvent n-heptane.

**Coating of the coating composition**

See Example 1.

**Preparation of the TTS**

See Example 1.

**EXAMPLE 5**

**Coating compositions**

The formulations of the fingolimod-containing coating composition are summarized below.

Table 5

| Ingredient (Trade Name) | Comparative Example E | | Example 5 | |
|---|---|---|---|---|
| | [g] | [%] | [g] | [%] |
| Fingolimod base | 0.15 | 3.0 | 0.15 | 3.0 |
| Povidone K90F (Kollidon™ 90 F from BASF) | 0.50 | 10.0 | 0.51 | 10.1 |
| 1-Dodecanol | - | - | 0.51 | 10.0 |
| Acrylic adhesive in ethyl acetate and hexane; Solids content of 41.2 % by weight (DURO-TAK™ 387-2510 from Henkel) | 10.57 | 87.0 | 9.34 | 76.9 |
| n-heptane | 1.29 | - | 2.01 | - |
| Total | 12.51 | 100.0 | 12.52 | 100.0 |
| Area Weight [g/m$^2$] | 110.6 | | 102.3 | |
| Loading API [$\mu$g/cm$^2$] | 336.2 | | 309.2 | |

**Preparation of the coating composition**

Fingolimod base was put in a suitable mixing vessel (beaker) and n-heptane was added. The pressure-sensitive adhesive (acrylic adhesive in ethyl acetate and hexane, having a solid content of 41.2 % by weight, DURO-TAK 387-2510 purchased from Henkel) was added to the slurry. Polyvinylpyrrolidone (Kollidon™ 90 F from BASF) was added to the mixture while stirring and the mass was stirred for approx. 3 hours.

For Example 5, 1-Dodecanol (purchased from Alfa Aesar) was added before n-heptane was added.

**Coating of the coating composition**

See Example 1

**Preparation of the TTS**

See Example 1.

**EXAMPLE 6**

**Measurement of skin permeation**

The permeated amount of fingolimod and the corresponding skin permeation rates of TTS prepared according to Examples 1 to 5 were determined by in vitro experiments in accordance with the OECD Guideline (adopted April 13, 2004) carried out with a 7.0 ml Franz diffusion cell. Split thickness human skin from cosmetic surgeries (female abdomen, date of birth 1996) was used. A dermatome was used to prepare skin to a thickness of 500 $\mu$m, with an intact epidermis for all TTS. Die cuts with an area of 1.16 cm$^2$ were punched from the TTS. The permeated amount of fingolimod in the receptor medium of the Franz diffusion cell (phosphate buffer solution pH 5.5 with 0.1 % Methyl-$\beta$-Cyclodextrine and 0.1 % saline azide as antibacteriological agent) at a temperature of 32 $\pm$ 1 °C was measured and the corresponding cumulative permeated amount and the skin permeation rate were calculated.

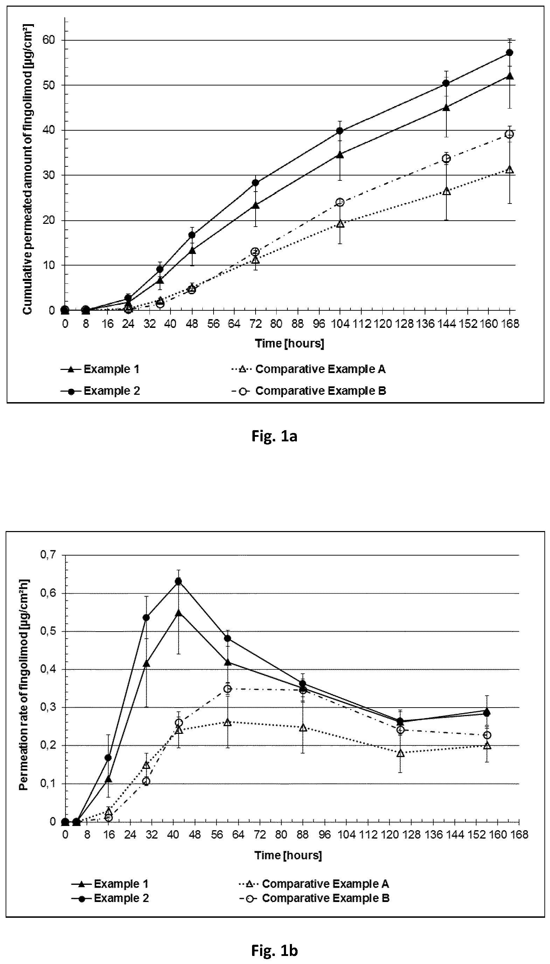

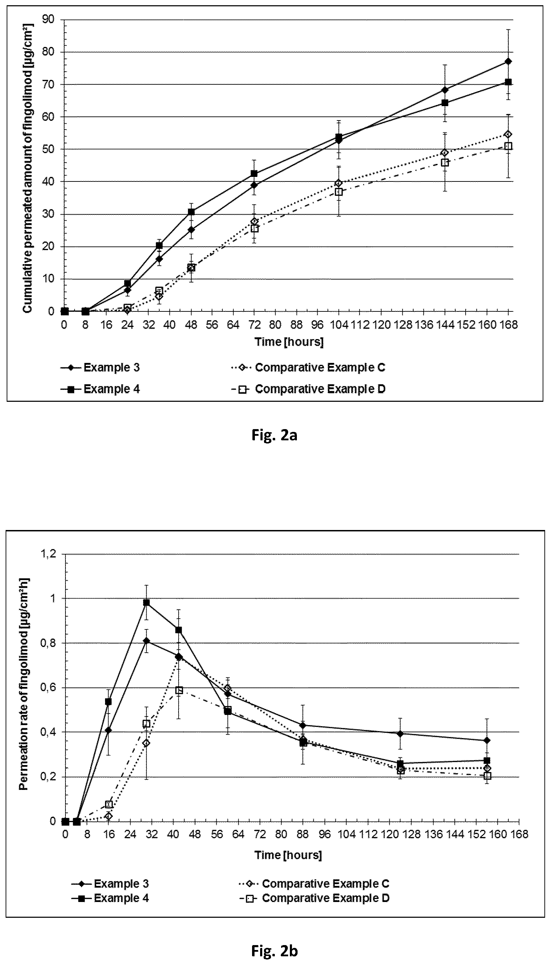

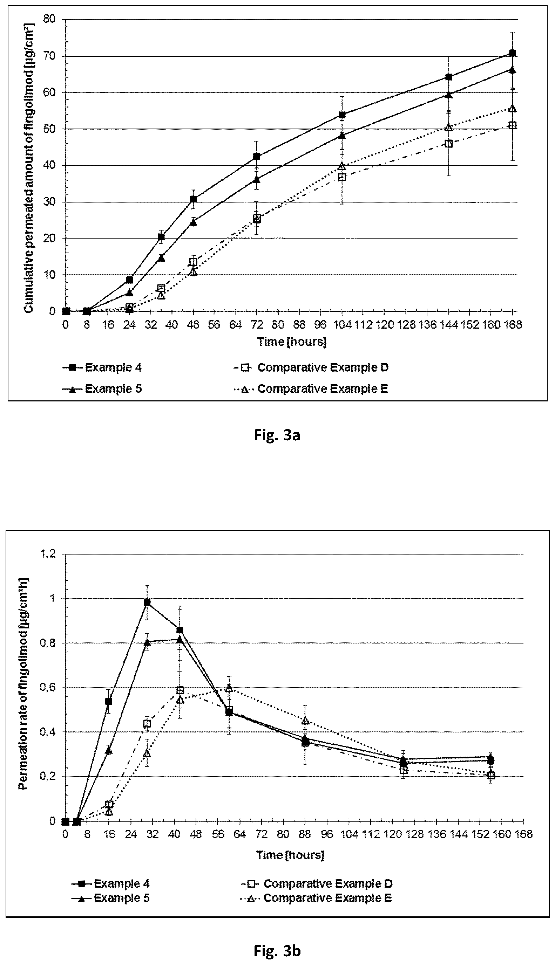

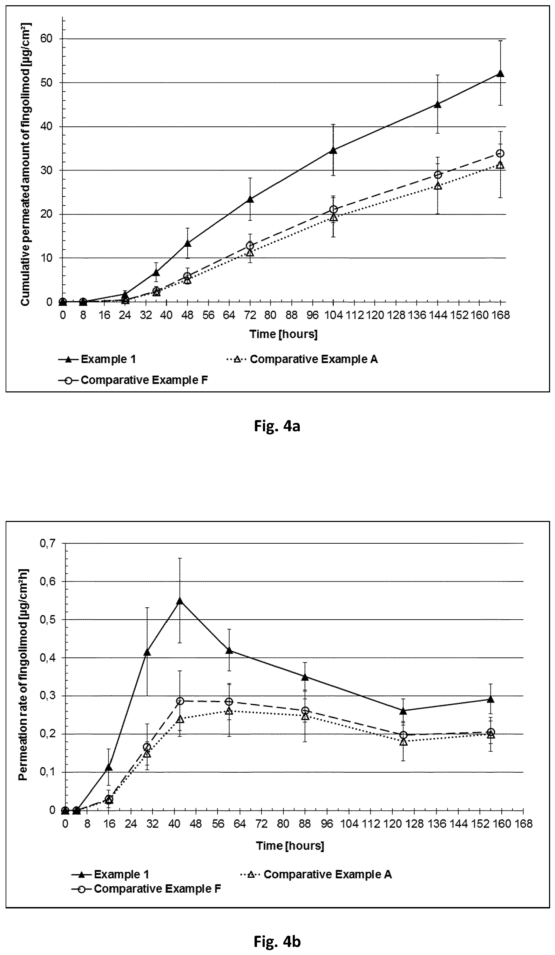

The results are shown in Tables 6-1 to 6-4 below and in Figures 1a to 4b,

Table 6-1

| Permeated amount with SD [$\mu$g/cm$^2$] | | | | | | | | |
|---|---|---|---|---|---|---|---|---|
| Elapsed time [h] | Comp. Ex. A (n = 3) | | Example 1 (n = 3) | | Comp. Ex. B (n = 3) | | Example 2 (n = 3) | |
| | Mean | SD | Mean | SD | Mean | SD | Mean | SD |
| 0 | 0 | 0 | 0 | 0 | 0 | 0 | 0 | 0 |

(continued)

| Permeated amount with SD [$\mu$g/cm$^2$] | | | | | | | | |
|---|---|---|---|---|---|---|---|---|
| Elapsed time [h] | Comp. Ex. A (n = 3) | | Example 1 (n = 3) | | Comp. Ex. B (n = 3) | | Example 2 (n = 3) | |
| | Mean | SD | Mean | SD | Mean | SD | Mean | SD |
| 8 | 0 | 0 | 0 | 0 | 0 | 0 | 0 | 0 |
| 24 | 0.46 | 0.18 | 1.81 | 0.77 | 0.19 | 0.02 | 2.66 | 0.97 |
| 36 | 1.79 | 0.36 | 4.99 | 1.39 | 1.28 | 0.14 | 6.43 | 0.67 |
| 48 | 2.89 | 0.57 | 6.60 | 1.33 | 3.12 | 0.17 | 7.57 | 0.12 |
| 72 | 6.27 | 1.64 | 10.1 | 1.31 | 8.37 | 0.35 | 11.6 | 0.49 |
| 104 | 7.93 | 2.19 | 11.2 | 1.20 | 11.1 | 0.59 | 11.6 | 0.37 |
| 144 | 7.22 | 2.07 | 10.4 | 1.26 | 9.66 | 0.63 | 10.5 | 0.97 |
| 168 | 4.79 | 1.07 | 7.01 | 0.94 | 5.43 | 0.55 | 6.80 | 0.17 |
| Cum. at 168 h | 31.3 | 7.6 | 52.1 | 7.4 | 39.1 | 1.8 | 57.2 | 3.0 |

| Permeated amount with SD [$\mu$g/cm$^2$] | | | | | | | | |
|---|---|---|---|---|---|---|---|---|
| Elapsed time [h] | Comp. Ex. C (n = 3) | | Example 3 (n = 3) | | Comp. Ex. D (n = 3) | | Example 4 (n = 3) | |
| | Mean | SD | Mean | SD | Mean | SD | Mean | SD |
| 0 | 0 | 0 | 0 | 0 | 0 | 0 | 0 | 0 |
| 8 | 0 | 0 | 0 | 0 | 0 | 0 | 0 | 0 |
| 24 | 0.38 | 0.33 | 6.54 | 1.79 | 1.23 | 0.13 | 8.60 | 0.86 |
| 36 | 4.21 | 1.94 | 9.72 | 0.63 | 5.26 | 0.39 | 11.8 | 0.94 |
| 48 | 8.83 | 2.08 | 8.91 | 0.72 | 7.09 | 1.59 | 10.3 | 1.07 |
| 72 | 14.3 | 1.12 | 13.7 | 1.52 | 12.0 | 2.67 | 11.8 | 1.73 |
| 104 | 11.8 | 0.62 | 13.8 | 2.88 | 11.3 | 3.06 | 11.4 | 1.10 |
| 144 | 9.47 | 0.55 | 15.7 | 2.81 | 9.18 | 1.47 | 10.4 | 0.98 |
| 168 | 5.73 | 0.29 | 8.69 | 2.36 | 4.99 | 0.89 | 6.54 | 0.81 |
| Cum. at 168 h | 54.7 | 5.9 | 77.1 | 9.9 | 51.1 | 9.8 | 70.8 | 5.6 |

| Permeated amount with SD [$\mu$g/cm$^2$] | | | | | | |
|---|---|---|---|---|---|---|
| Elapsed time [h] | Comp. Ex. E (n = 3) | | Example 5 (n = 3) | | Comp. Ex. F (n = 3) | |
| | Mean | SD | Mean | SD | Mean | SD |
| 0 | 0 | 0 | 0 | 0 | 0 | 0 |
| 8 | 0 | 0 | 0 | 0 | 0 | 0 |
| 24 | 0.73 | 0.31 | 5.12 | 0.33 | 0.49 | 0.37 |
| 36 | 3.67 | 0.73 | 9.66 | 0.45 | 2.00 | 0.71 |
| 48 | 6.57 | 0.46 | 9.82 | 1.77 | 3.45 | 0.93 |
| 72 | 14.3 | 1.27 | 11.8 | 1.83 | 6.85 | 1.15 |
| 104 | 14.5 | 2.09 | 12.0 | 1.18 | 8.36 | 0.97 |
| 144 | 10.8 | 1.39 | 11.20 | 1.53 | 7.89 | 1.05 |
| 168 | 5.20 | 0.86 | 6.94 | 0.32 | 4.91 | 0.70 |
| Cum. at 168 h | 55.8 | 5.0 | 66.5 | 5.3 | 34.0 | 2.1 |

Table 6-2

| Skin permeation rate with SD [$\mu$g/cm$^2$-hr] | | | | | | | |
|---|---|---|---|---|---|---|---|
| Elapsed time [h] | Comp. Ex. A (n = 3) | | Example 1 (n = 3) | | Comp. Ex. B (n = 3) | | Example 2 (n = 3) | |
| | Rate | SD | Rate | SD | Rate | SD | Rate | SD |
| 0 | 0 | 0 | 0 | 0 | 0 | 0 | 0 | 0 |
| 4 | 0 | 0 | 0 | 0 | 0 | 0 | 0 | 0 |
| 16 | 0.03 | 0.01 | 0.11 | 0.05 | 0.01 | 0.00 | 0.17 | 0.06 |
| 30 | 0.15 | 0.03 | 0.42 | 0.12 | 0.11 | 0.01 | 0.54 | 0.06 |
| 42 | 0.24 | 0.05 | 0.55 | 0.11 | 0.26 | 0.01 | 0.63 | 0.01 |
| 60 | 0.26 | 0.07 | 0.42 | 0.05 | 0.35 | 0.01 | 0.48 | 0.02 |
| 88 | 0.25 | 0.07 | 0.35 | 0.04 | 0.35 | 0.02 | 0.36 | 0.01 |
| 124 | 0.18 | 0.05 | 0.26 | 0.03 | 0.24 | 0.02 | 0.26 | 0.02 |
| 156 | 0.20 | 0.04 | 0.29 | 0.04 | 0.23 | 0.02 | 0.28 | 0.01 |
| Skin permeation rate with SD [$\mu$g/cm$^2$-hr] | | | | | | | |
| Elapsed time [h] | Comp. Ex. C (n = 3) | | Example 3 (n = 3) | | Comp. Ex. D (n = 3) | | Example 4 (n = 3) | |
| | Rate | SD | Rate | SD | Rate | SD | Rate | SD |
| 0 | 0 | 0 | 0 | 0 | 0 | 0 | 0 | 0 |
| 4 | 0 | 0 | 0 | 0 | 0 | 0 | 0 | 0 |
| 16 | 0.02 | 0.02 | 0.41 | 0.11 | 0.08 | 0.01 | 0.54 | 0.05 |
| 30 | 0.35 | 0.16 | 0.81 | 0.05 | 0.44 | 0.03 | 0.98 | 0.08 |
| 42 | 0.74 | 0.17 | 0.74 | 0.06 | 0.59 | 0.13 | 0.86 | 0.09 |
| 60 | 0.60 | 0.05 | 0.57 | 0.06 | 0.50 | 0.11 | 0.49 | 0.07 |
| 88 | 0.37 | 0.02 | 0.43 | 0.09 | 0.35 | 0.10 | 0.36 | 0.03 |
| 124 | 0.24 | 0.01 | 0.39 | 0.07 | 0.23 | 0.04 | 0.26 | 0.02 |
| 156 | 0.24 | 0.01 | 0.36 | 0.10 | 0.21 | 0.04 | 0.27 | 0.03 |
| Skin permeation rate with SD [$\mu$g/cm$^2$-hr] | | | | | | | |
| Elapsed time [h] | Comp. Ex. E (n = 3) | | Example 5 (n = 3) | | Comp. Ex. F (n = 3) | | | |
| | Rate | SD | Rate | SD | Rate | SD | | |
| 0 | 0 | 0 | 0 | 0 | 0 | 0 | | |
| 4 | 0 | 0 | 0 | 0 | 0 | 0 | | |
| 16 | 0.05 | 0.02 | 0.32 | 0.02 | 0.03 | 0.02 | | |
| 30 | 0.31 | 0.06 | 0.81 | 0.04 | 0.17 | 0.06 | | |
| 42 | 0.55 | 0.04 | 0.82 | 0.15 | 0.29 | 0.08 | | |
| 60 | 0.60 | 0.05 | 0.49 | 0.08 | 0.29 | 0.05 | | |
| 88 | 0.45 | 0.07 | 0.37 | 0.04 | 0.26 | 0.03 | | |
| 124 | 0.27 | 0.03 | 0.28 | 0.04 | 0.20 | | 0.03 | |
| 156 | 0.22 | 0.04 | 0.29 | 0.01 | 0.21 | | 0.03 | |

Table 6-3

| Ratio Mean Cumulative permeated amount at 168 hours Example / Comparative Example | | | | | |
|---|---|---|---|---|---|
| Ex. 1/ Comp. Ex. A | Ex. 2/ Comp. Ex. B | Ex. 3/ Comp. Ex. C | Ex. 4/ Comp. Ex. D | Ex. 5/ Comp. Ex. E | Ex. 1/ Comp. Ex. F |
| 1.7 | 1.5 | 1.4 | 1.4 | 1.2 | 1.5 |

Table 6-4

| Ratio Mean Cumulative permeated amount at 168 hours / API Loading Example / Comparative Example (Ratio Active agent utilization) | | | | | |
|---|---|---|---|---|---|
| Ex. 1/ Comp. Ex. A | Ex. 2/ Comp. Ex. B | Ex. 3/ Comp. Ex. C | Ex. 4/ Comp. Ex. D | Ex. 5/ Comp. Ex. E | Ex. 1/ Comp. Ex. F |
| 1.7 | 1.5 | 1.4 | 1.4 | 1.3 | 1.5 |

**EXAMPLES 7-1 AND 7-2**

**Coating compositions**

The formulations of the fingolimod-containing coating composition are summarized below.

Table 7

| Ingredient (Trade Name) | Example 7-1 | | Example 7-2 | |
|---|---|---|---|---|
| | [g] | [%] | [g] | [%] |
| Fingolimod base | 0.15 | 2.95 | 0.15 | 3.0 |
| Ethylcellulose | 0.05 | 1.0 | 0.05 | 1.0 |
| 1-Dodecanol | 0.52 | 10.05 | 0.36 | 7.0 |
| Pressure-sensitive adhesive based on polyisobutylenes in n-heptane/n-hexane Solids content of 41.28 % by weight (Oppanol™ B10/N100 in a ratio of 85/15 from BASF) | 10.59 | 86.0 | 10.75 | 89.0 |
| n-hexane | 1.38 | - | 1.17 | - |
| Total | 12.69 | 100.0 | 12.48 | 100.0 |
| Area Weight [g/m$^2$] | 101.2 | | 98.5 | |
| Loading API [$\mu$g/cm$^2$] | 297.8 | | 296.3 | |

**Preparation of the coating composition**

Fingolimod base was put in a suitable mixing vessel (beaker) and 1-Dodecanol was added. The solvent n-hexane is added to the slurry and Ethylcellulose (purchased from IMCD) was added to the mixture while stirring. The mass was stirred for approx. 1.5 hours. The pressure-sensitive adhesive (based on polyisobutylenes in the form of a mixture in n-heptane, having a solid content of 41.28 % by weight, Oppanol™ B10/N100 in a ratio of 85/15 from BASF) was added and the mass was stirred for approx. 1 hour.

**Coating of the coating composition**

See Example 1.

**Preparation of the TTS**

See Example 1.

**EXAMPLE 8**

**Measurement of skin permeation**

The permeated amount of fingolimod and the corresponding skin permeation rates of TTS prepared according to Examples 7-1 and 7-2 were determined by in vitro experiments in accordance with the OECD Guideline (adopted April 13, 2004) carried out with a 7.0 ml Franz diffusion cell. Split thickness human skin from cosmetic surgeries (female abdomen, date of birth 1991) was used. A dermatome was used to prepare skin to a thickness of 500 $\mu$m, with an intact epidermis for all TTS. Die cuts with an area of 1.16 cm$^2$ were punched from the TTS. The permeated amount of fingolimod in the receptor medium of the Franz diffusion cell (phosphate buffer solution pH 5.5 with 0.1 % Methyl-$\beta$-Cyclodextrine and 0.1 % saline azide as antibacteriological agent) at a temperature of 32 $\pm$ 1 °C was measured and the corresponding cumulative permeated amount and the skin permeation rate were calculated.

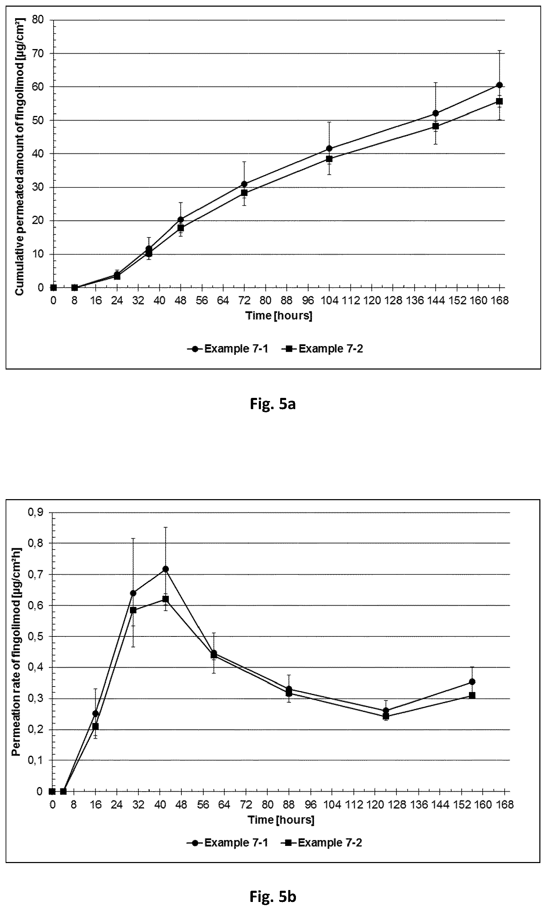

The results are shown in Table 8-1 and Table 8-2 below and in Figures 5a and 5b.

Table 8-1

| Permeated amount with SD [$\mu$g/cm$^2$] | | | | |
|---|---|---|---|---|
| Elapsed time [h] | Example 7-1 (n = 3) | | Example 7-2 (n = 3) | |
| | Mean | SD | Mean | SD |
| 0 | 0 | 0 | 0 | 0 |
| 8 | 0 | 0 | 0 | 0 |
| 24 | 4.02 | 1.28 | 3.36 | 0.49 |
| 36 | 7.69 | 2.10 | 7.01 | 0.61 |
| 48 | 8.61 | 1.62 | 7.44 | 0.22 |
| 72 | 10.72 | 1.57 | 10.54 | 0.34 |
| 104 | 10.61 | 1.38 | 10.19 | 0.33 |
| 144 | 10.44 | 1.28 | 9.69 | 0.21 |
| 168 | 8.50 | 1.14 | 7.44 | 0.27 |
| Cum. at 168 h | **60.6** | **10.3** | **55.67** | **1.68** |

Table 8-2

| Skin permeation rate with SD [$\mu$g/cm$^2$-hr] | | | | |
|---|---|---|---|---|
| Elapsed time [h] | Example 7-1 (n = 3) | | Example 7-2 (n = 3) | |
| | Rate | SD | Rate | SD |
| 0 | 0 | 0 | 0 | 0 |
| 4 | 0 | 0 | 0 | 0 |
| 16 | 0.25 | 0.08 | 0.21 | 0.03 |
| 30 | 0.64 | 0.17 | 0.58 | 0.05 |
| 42 | 0.72 | 0.13 | 0.62 | 0.02 |
| 60 | 0.45 | 0.07 | 0.44 | 0.01 |
| 88 | 0.33 | 0.04 | 0.32 | 0.01 |
| 124 | 0.26 | 0.03 | 0.24 | 0.01 |
| 156 | 0.35 | 0.05 | 0.31 | 0.01 |

## EXAMPLE 9

**Coating compositions**

The formulations of the fingolimod-containing coating composition are summarized below.

Table 9-1

| Ingredient (Trade Name) | Ex. 9-1 | | Ex. 9-2 | | Ex. 9-3 | | Ex. 9-4 | |
|---|---|---|---|---|---|---|---|---|
| | Amt [g] | Solids [%] | Amt [g] | Solids [%] | Amt [g] | Solids [%] | Amt [g] | Solids [%] |
| Fingolimod base | 1.50 | 7.5 | 1.50 | 7.5 | 1.51 | 7.5 | 1.51 | 7.5 |
| 1-Dodecanol | 3.04 | 15.0 | 3.06 | 15.0 | 3.04 | 15.0 | - | - |
| Povidone K90F (Kollidon™ 90 F from BASF) | - | - | 1.00 | 5.0 | 1.00 | 5.0 | | |
| Acrylic adhesive in ethyl acetate, ethanol, n-heptane and methanol. Solids content of 42.8 % by weight (DURO-TAK™ 387-2516) | 36.22 | 77.5 | - | - | - | - | - | - |
| Pressure-sensitive adhesive based on polyisobutylenes in n-heptane/n-hexane Solids content of 41.28 % by weight (Oppanol™ B10/N100 in a ratio of 85/15 from BASF) | - | - | 35.51 | 72.5 | - | - | - | - |
| Silicone acrylic hybrid PSA in ethyl acetate Solids content of 50 % by weight (SilAc-PSA 7-6302 from Dow Coming Healthcare) | - | - | - | - | 28.55 | 72.5 | 26.71 | 67.5 |
| Lauryl lactate | - | - | - | - | - | - | 2.03 | 10.0 |
| Polyvinyl caprolactam-polyvinyl acetate-polyethylene glycol graft (PCL-PVAc-PEG, Soluplus™) | - | - | - | - | - | - | 2.00 | 10.0 |
| 1,2-Propandiol | - | - | - | - | - | - | 1.01 | 5.0 |
| Ethylacetate | 16.41 | - | 8.93 | - | 23.50 | - | 11.23 | - |
| Total | 57.17 | 100.00 | 50.00 | 100.00 | 57.60 | 100.00 | 44.49 | 100.00 |
| Area Weight [g/m$^2$] | 151.2 | | 151.2 | | 154.1 | | 157.1 | |
| Loading API [μg/cm$^2$] | 1131.4 | | 1133.1 | | 1157.1 | | 1179.2 | |

**Preparation of the coating composition**

For Example 9-1: Fingolimod base is put in a suitable mixing vessel (beaker) and 1-Dodecanol is added. Solvent ethylacetate is added to the slurry and the pressure-sensitive adhesive (acrylic adhesive in ethyl acetate, ethanol, n-heptane and methanol, having a solids content of 42.8 % by weight (DURO-TAK™ 387-2516 purchased from Henkel) was added to the slurry. The mass was stirred for approx. 3 hours.

For Example 9-2: Fingolimod base is put in a suitable mixing vessel (beaker) and 1-Dodecanol is added. Solvent ethylacetate is added to the slurry and the pressure-sensitive adhesive (based on polyisobutylenes in the form of a mixture in n-heptane, having a solid content of 41.28 % by weight, Oppanol™ B10/N100 in a ratio of 85/15 from BASF) was added. Polyvinylpyrrolidone (KollidonTM 90 F from BASF) was added to the mixture while stirring and the mass was stirred for approx. 4 hours.

For Example 9-3: Fingolimod base is put in a suitable mixing vessel (beaker) and 1-Dodecanol is added. Solvent ethylacetate is added to the slurry and the pressure-sensitive adhesive (Silicone acrylic hybrid PSA in ethyl acetate, solids content of 50 % by weight, SilAc-PSA 7-6302 from Dow Corning Healthcare) was added. Polyvinylpyrrolidone (KollidonTM 90 F from BASF) was added to the mixture while stirring and the mass was stirred for approx. 3.5 hours.

For Example 9-4: Fingolimod base is put in a suitable mixing vessel (beaker) and lauryl lactate is added. 1,2-Propandiol as well as solvent ethylacetate are added to the slurry. Polyvinyl caprolactam-polyvinyl acetate-polyethylene glycol graft (PCL-PVAc-PEG, Soluplus™ )was added to the mixture while stirring and the mass was stirred for approx. 1 hour. The pressure-sensitive adhesive (Silicone acrylic hybrid PSA in ethyl acetate, solids content of 50 % by weight, SilAc-PSA 7-6302 from Dow Corning Healthcare) was added and the mass was stirred for approx. 3.5 hours.

**Coating of the coating composition**

The fingolimod base-containing coating composition was coated within 24 hours on a abhesively equipped polyethylenterephthalate (PET) foil which may function as a release liner and dried at room temperature and 60 °C for 15 minutes each.

The coating thickness was chosen such that removal of the solvents results in an area weight of the matrix layer of approx. 150 g/m$^2$. The dried film was then laminated with a backing layer (polyethylenterephthalate (PET) foil 19 $\mu$m) to provide the fingolimod-containing self-adhesive layer structure.

**Preparation of the TTS**

See Example 1.

***In vivo* study using *Goettingen* minipigs**

In a first stage, the metabolism of fingolimod to fingolimod phosphate was investigated in *Goettingen* minipigs (male, about 3-4 months), after oral administration of 0.5 mg fingolimod per day (Gilenya™ capsule) for seven days to show the suitability of the study model.

One *Goettingen* minipig was used for one daily dose of 0.5 mg fingolimod.

The study took place in an animal room provided with filtered air at a temperature of 21 °C $\pm$3 °C. The temperature and relative humidity in the animal room was recorded hourly during the study and the records were retained. An SDS minipig diet (SMP (E) SQC) from Special Diets Services, Witham Essex, CM8 3AD, U.K., was offered twice daily in an amount of approximately 125 g per animal per meal.

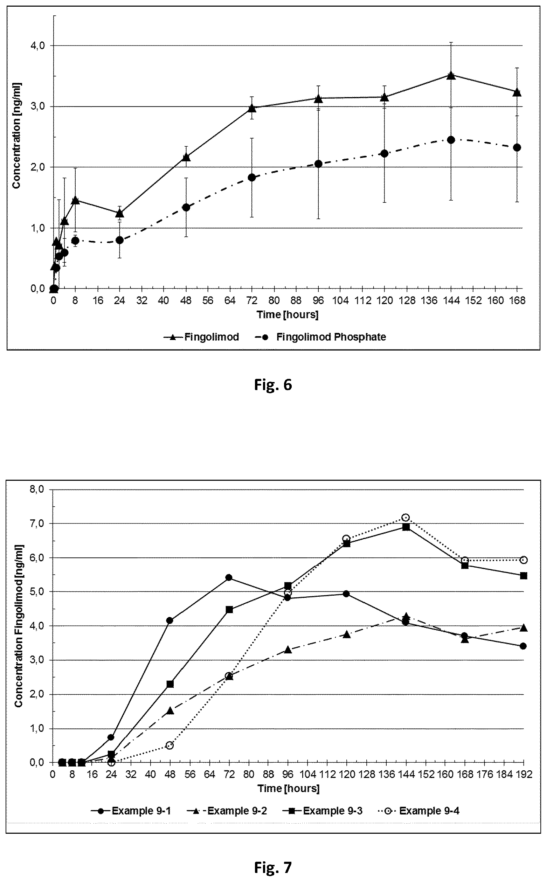

3 ml blood samples were taken at 0 hours, 0.5 hours, 1 hours, 2 hours, 4 hours, 8 hours, 24 hours, 48 hours, 72 hours, 96 hours, 120 hours, 144 hours, 168 hours. The analysis was performed with a qualified LC-MS/MS method for the measurement of analytes in minipig K3EDTA whole blood. AUC values were calculated from the whole blood concentration. The results are shown in Tables 9-2 and 9-3, and Figure 6.

Table 9-2

| Blood Concentration [ng/ml] (Gilenya™ capsule) | | | | |
|---|---|---|---|---|
| **Elapsed time [h]** | **Fingolimod (n = 3)** | | **Fingolimod Phosphate (n = 3)** | |
| | **Mean** | **SD** | **Mean** | **SD** |
| **0** | BLQ | n.a. | BLQ | n.a. |
| **0.5** | 0.385 | 0.000 | BLQ | n.a. |
| **1** | 0.782 | 0.000 | 0.345 | 0.000 |
| **2** | 0.718 | 0.747 | 0.534 | 0.000 |
| **4** | 1.129 | 0.694 | 0.600 | 0.231 |
| **8** | 1.463 | 0.525 | 0.790 | 0.094 |
| **24** | 1.250 | 0.113 | 0.803 | 0.295 |
| **48** | 2.173 | 0.171 | 1.342 | 0.482 |
| **72** | 2.980 | 0.183 | 1.830 | 0.653 |
| **96** | 3.137 | 0.203 | 2.057 | 0.909 |

(continued)

| Blood Concentration [ng/ml] (Gilenya™ capsule) | | | | |
|---|---|---|---|---|
| Elapsed time [h] | Fingolimod (n = 3) | | Fingolimod Phosphate (n = 3) | |
| | Mean | SD | Mean | SD |
| 120 | 3.157 | 0.187 | 2.230 | 0.813 |
| 144 | 3.523 | 0.538 | 2.453 | 0.997 |
| 168 | 3.243 | 0.395 | 2.330 | 0.904 |
| $AUC_{(0-168)}$ [(ng/ml) h] | 443 | 21 | 292 | 108 |
| $C_{max}$ [ng/ml] | 3.60 | 0.41 | 2.46 | 0.98 |
| BLQ: below limit of quantification. | | | | |

Table 9-3

| Ratio Mean $C_{max}$ Fingolimod Phosphate/ Mean $C_{max}$ Fingolimod |
|---|
| 0.68 |

For purposes of comparison, the ratio of $C_{max}$ fingolimod phosphate / $C_{max}$ fingolimod in humans at steady state after administration of 0.5 mg/day is about 0.5. Steady-state exposure is reached between 1 to 2 months during once-daily dosing with an estimated 11-fold accumulation of blood levels from first dose to steady state.

In a second stage, the TTS prepared according to Examples 9-1 to 9-4 were tested in an *in vivo* study (randomized by simple random sample method) using the *Goettingen* minipigs (male, about 6-7 months). Diecuts with an area of 10 cm$^2$ were punched from the TTS and one *Goettingen* minipig was used for one TTS formulation. Seven drug containing TTS and two placebo TTS (each 10 cm$^2$) were used per minipig. The total wear time of all 9 patches per minipig (7 active and 2 placebo) patches was 168 hours.

The study took place in an animal room provided with filtered air at a temperature of 21 ± 3 °C. The temperature and relative humidity in the animal room were recorded hourly during the study and the records were retained. An SDS minipig diet (SMP (E) SQC) from Special Diets Services, Witham Essex, CM8 3AD, U.K., was offered twice daily. From arrival and until first feeding on Day 1, the amount of diet was approximately 125 g per animal per meal; thereafter the animals received 150 g diet per meal.

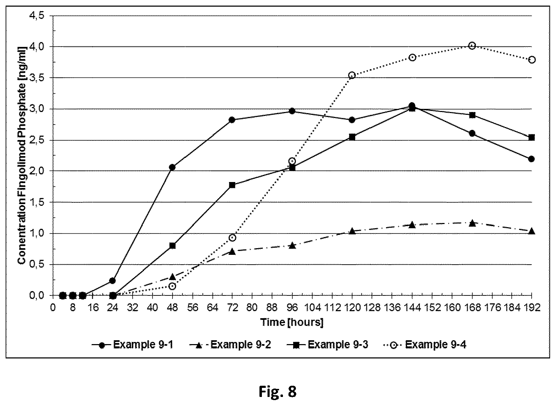

Following the above single dose application of the TTS (7 active and 2 placebo, each 10 cm$^2$), 3 ml blood samples were taken at 0 hours, 4 hours, 8 hours, 12 hours, 24 hours, 48 hours, 72 hours, 96 hours, 120 hours, 144 hours, 168 hours (patch removal), and 192 hours. The analysis was performed with a qualified LC-MS/MS method for the measurement of analytes in minipig K3EDTA whole blood. AUC values were calculated from the whole blood concentration. The results are shown in Tables 9-4 to 9-6, and Figures 7 and 8.

Table 9-4

| Fingolimod Blood Concentrations [ng/ml] | | | | |
|---|---|---|---|---|
| Elapsed time [h] | Ex. 9-1 | Ex. 9-2 | Ex. 9-3 | Ex. 9-4 |
| 0 | BLQ | BLQ | BLQ | BLQ |
| 4 | BLQ | BLQ | BLQ | BLQ |
| 8 | BLQ | BLQ | BLQ | BLQ |
| 12 | BLQ | BLQ | BLQ | BLQ |
| 24 | 0.72 | 0.13 | 0.25 | BLQ |
| 48 | 4.15 | 1.53 | 2.30 | 0.50 |
| 72 | 5.40 | 2.54 | 4.47 | 2.53 |
| 96 | 4.81 | 3.31 | 5.17 | 4.96 |

(continued)

| Fingolimod Blood Concentrations [ng/ml] | | | | |
|---|---|---|---|---|
| Elapsed time [h] | Ex. 9-1 | Ex. 9-2 | Ex. 9-3 | Ex. 9-4 |
| 120 | 4.93 | 3.76 | 6.42 | 6.55 |
| 144 | 4.09 | 4.29 | 6.90 | 7.18 |
| 168 | 3.70 | 3.62 | 5.77 | 5.92 |
| 192 | 3.40 | 3.96 | 5.47 | 5.94 |
| $AUC_{(0-168)}$ [(ng/ml) h] | 619 | 416 | 680 | 592 |
| $C_{max}$ [ng/ml] | 5.40 | 4.29 | 6.90 | 7.18 |
| BLQ: below limit of quantification. | | | | |

Table 9-5

| Fingolimod Phosphate Blood Concentrations [ng/ml] | | | | |
|---|---|---|---|---|
| Elapsed time [h] | Ex. 9-1 | Ex. 9-2 | Ex. 9-3 | Ex. 9-4 |
| 0 | BLQ | BLQ | BLQ | BLQ |
| 4 | BLQ | BLQ | BLQ | BLQ |
| 8 | BLQ | BLQ | BLQ | BLQ |
| 12 | BLQ | BLQ | BLQ | BLQ |
| 24 | 0.24 | BLQ | BLQ | BLQ |
| 48 | 2.06 | 0.30 | 0.80 | 0.15 |
| 72 | 2.82 | 0.71 | 1.78 | 0.93 |
| 96 | 2.96 | 0.81 | 2.06 | 2.16 |
| 120 | 2.82 | 1.04 | 2.55 | 3.54 |
| 144 | 3.05 | 1.14 | 3.01 | 3.83 |
| 168 | 2.60 | 1.17 | 2.90 | 4.02 |
| 192 | 2.19 | 1.04 | 2.54 | 3.79 |
| $AUC_{(0-168)}$ [(ng/ml) h] | 365 | 110 | 280 | 303 |
| $C_{max}$ [ng/ml] | 3.05 | 1.17 | 3.01 | 4.02 |
| BLQ: below limit of quantification. | | | | |

Table 9-6

| Ratio $C_{max}$ Fingolimod phosphate/ $C_{max}$ Fingolimod | | | |
|---|---|---|---|
| Ex. 9-1 (n = 3) | Ex. 9-2 (n = 3) | Ex. 9-3 (n = 3) | Ex. 9-4 (n = 3) |
| 0.56 | 0.27 | 0.44 | 0.56 |

After removal of the TTS, the skin condition was macroscopically determined and a Draize score obtained based on the score scheme below. Histopathological examination of the epidermis and the dermis revealed minimal to mild/moderate local irritation. The residual amount of fingolimod was determined in the removed TTS by quantitative HPLC and the dermally delivered amount of fingolimod calculated as the difference to the initial amount of fingolimod included in the TTS. The results are shown in Table 10.

Table 10

| Values | Ex. 9-1 | Ex. 9-2 | Ex. 9-3 | Ex. 9-4 |
|---|---|---|---|---|
| Histopathological examination | Minimal to mild local irritation | Minimal to mild local irritation | Minimal to mild local irritation | Minimal to moderate local irritation |
| Draize* score erythema/edema (7 verum / 2 placebo) at 168 hours | Verum: 0/0; 2/0; 2/0; 2/0; 1/0; 2/0; 2/0 <br> Placebo: 0/0; 0/0 | Verum: 1/0; 0/0; 0/0; 1/0; 1/0; 1/0; 1/0; <br> Placebo: 0/0; 0/0 | Verum: 2/0; 1/0; 1/0; 1/0; 2/0; 2/0; 2/0 <br> Placebo: 1/0; 0/0 | Verum: 2/0; 2/0; 2/0; 2/0; 1/0; 2/0; 1/0 <br> Placebo: 0/0; 0/0 |
| Draize* score erythema/edema (7 verum / 2 placebo) at 192 hours | Verum: 0/0; 2/0; 1/1; 2/0; 1/0; 2/0; 2/0 <br> Placebo: 0/0; 0/0 | Verum: 1/1; 0/0; 1/0; 1/0; 1/0; 1/0; 1/0; <br> Placebo: 0/0; 0/0 | Verum: 3/0; 0/0; 1/0; 2/0; 1/0; 1/0; 1/0 <br> Placebo: 0/0; 0/0 | Verum: 2/1; 1/0; 1/0; 2/0; 1/0; 2/0; 0/0 <br> Placebo: 0/0; 0/0 |
| Amount of fingolimod dermally delivered after 168 hours [%] | 34 ± 3.3 | 41 ± 8.1 | 26 ±9.8 | 45 ± 6.2 |

*: Score schemes for the evaluation of skin irritation potential according to Draize:
0 = No erythema, no edema, 1 = Very slight erythema (barely perceptible), very slight edema (barely perceptible), 2 = Well-defined erythema, Slight edema, 3 = Moderate to severe erythema, moderate edema, 4 = Severe erythema, severe edema.

**Claims**

1. A transdermal therapeutic system for the transdermal administration of fingolimod comprising a fingolimod-containing layer structure,
   the fingolimod-containing layer structure comprising:

   A) a backing layer, and
   B) a fingolimod-containing layer comprising:

   a) a therapeutically effective amount of fingolimod,
   b) at least one polymer, and
   c) dodecan-1-ol,
   wherein the weight ratio of dodecan-1-ol : fingolimod ranges from 1.5 : 1 to 5 : 1.

2. The transdermal therapeutic system according to claim 1, wherein the at least one polymer is selected from the group consisting of a silicone acrylic hybrid polymer, a polymer based on polysiloxanes, a polymer based on polyisobutylenes, and an acrylate polymer.

3. The transdermal therapeutic system according to claim 1 or 2, wherein the at least one polymer is contained in the fingolimod-containing layer in an amount of from about 40 % to about 99 % by weight, preferably of from about 50 % to about 99 % by weight, more preferably of from about 60 % to about 99 % by weight based on the fingolimod-containing layer.

4. The transdermal therapeutic system according to any one of claims 1 to 3,
   wherein the at least one polymer is a polymer-based pressure-sensitive adhesive.

5. The transdermal therapeutic system according to any one of claims 1 to 4, wherein the fingolimod-containing layer is a fingolimod-containing matrix layer.

6. The transdermal therapeutic system according to any one of claims 1 to 5, wherein the dodecan-1-ol is contained in an amount of from 2 % to 40 % by weight, preferably of from 2 % to 30 % by weight, more preferably of from 4 % to 20 % by weight based on the fingolimod-containing layer.

7. The transdermal therapeutic system according to any one of claims 1 to 6, wherein the fingolimod is contained in an amount of from 1 % to 20 % by weight, preferably of from 1 % to 15 % by weight, more preferably of from 2 % to 10 % by weight based on the fingolimod-containing layer.

8. The transdermal therapeutic system according to any one of claims 1 to 7, wherein the fingolimod-containing layer structure contains $0.1 \text{ mg/cm}^2$ to $2.0 \text{ mg/cm}^2$, preferably $0.1 \text{ mg/cm}^2$ to $1.5 \text{ mg/cm}^2$, more preferably $0.2 \text{ mg/cm}^2$ to $1.2 \text{ mg/cm}^2$ fingolimod based on the fingolimod-containing layer.

9. The transdermal therapeutic system according to any one of claims 1 to 8, wherein the fingolimod-containing layer is obtainable by coating and drying a fingolimod-containing coating composition, which comprises the at least one polymer, and the dodecan-1-ol and the therapeutically effective amount of fingolimod in a weight ratio of dodecan-1-ol: fingolimod of from 1.5 : 1 to 5 : 1.

10. The transdermal therapeutic system according to any one of claims 1 to 9, wherein the fingolimod-containing layer further comprises an auxiliary polymer, preferably selected from the group consisting of alkyl methacrylate copolymers, amino alkyl methacrylate copolymers, methacrylic acid copolymers, methacrylic ester copolymers, ammonioalkyl methacrylate copolymers, polyvinylpyrrolidones, vinylpyrrolidone-vinyl acetate copolymers, polyvinyl caprolactam-polyvinyl acetate-polyethylene glycol copolymer, cellulose derivatives, and mixtures thereof, more preferably selected from cellulose derivatives.

11. The transdermal therapeutic system according to claim 10, wherein the auxiliary polymer is contained in an amount of from about 0.5 % to about 20 %, preferably of from about 0.5 % to about 10 % by weight, more preferably of from about 1 % to about 5 % by weight by weight based on the fingolimod-containing layer.

12. The transdermal therapeutic system according to any one of claims 1 to 11, wherein the fingolimod-containing layer does not comprise a polyvinylpyrrolidone.

13. The transdermal therapeutic system according to any one of claims 1 to 12, wherein the fingolimod-containing layer does not comprise an ester of dodecanol, an organosulfur compound, and/or a fatty acid ester.

14. The transdermal therapeutic system according to any one of claims 1 to 13, which provides a mean release rate of fingolimod of 0.1 to 1.0 mg/day, preferably over at least 72 hours, about 84 hours, about 96 hours, or about 168 hours of administration.

15. The transdermal therapeutic system according to any one of claims 1 to 14, which provides a cumulative permeated amount of fingolimod of more than 1.5 $\mu\text{g/cm}^2$ within the first 24 hours of administration, and/or a cumulative permeated amount of fingolimod of more than 6.0 $\mu\text{g/cm}^2$ within the first 36 hours of administration, as measured in a Franz diffusion cell with dermatomed human skin.

16. The transdermal therapeutic system according to any one of claims 1 to 15, which provides a skin permeation rate of fingolimod of more than 0.1 $\mu\text{g/cm}^2$-hr at hour 16 after administration as measured in a Franz diffusion cell with dermatomed human skin.

17. The transdermal therapeutic system according to any one of claims 1 to 16, which provides a ratio of $C_{max}$ fingolimod phosphate : $C_{max}$ fingolimod of 0.2 : 1 to 0.8 : 1 over about 168 hours of administration after a single-dose administration to a subject population.

18. The transdermal therapeutic system according to any one of claims 1 to 17, for use in a method of treating an immune disorder, preferably multiple sclerosis.

19. A method of manufacture of a transdermal therapeutic system according to any one of claims 1 to 18 comprising the steps of:

    1) providing a fingolimod-containing coating composition comprising

        a) fingolimod,
        b) at least one polymer,

c) dodecan-1-ol, and
d) optionally a solvent,

2) coating the fingolimod-containing coating composition onto a release liner in an amount to provide the desired area weight,
3) drying the coated fingolimod-containing coating composition to provide the fingolimod-containing layer,
4) laminating the fingolimod-containing layer to a backing layer to provide an fingolimod-containing layer structure,
5) optionally providing an additional skin contact layer by coating and drying an active agent-free coating composition or an active agent-containing coating composition according to steps 2 and 3, removing the release liner of the fingolimod-containing layer and laminating the adhesive side of the skin contact layer onto the adhesive side of the fingolimod-containing layer to provide an fingolimod-containing layer structure,
6) punching the individual systems from the fingolimod-containing layer structure,
7) optionally adhering to the individual systems an active agent-free self-adhesive layer structure comprising also a backing layer and an active agent-free pressure-sensitive adhesive layer and which is larger than the individual systems of the fingolimod-containing layer structure.

20. Use of dodecan-1-ol in a transdermal therapeutic system comprising fingolimod for reducing the lag time of the permeation of fingolimod,

**Patentansprüche**

1. Ein transdermales therapeutisches System zur transdermalen Verabreichung von Fingolimod, umfassend eine Fingolimod-enthaltende Schichtstruktur,
   wobei die Fingolimod-enthaltende Schichtstruktur umfasst:

   A) eine Rückschicht und
   B) eine Fingolimod-enthaltende Schicht, umfassend:

   a) eine therapeutisch wirksame Menge an Fingolimod,
   b) mindestens ein Polymer, und
   c) Dodecan-1-ol,
   wobei das Gewichtsverhältnis von Dodecan-1-ol : Fingolimod im Bereich von 1,5 : 1 bis 5 : 1 liegt.

2. Transdermales therapeutisches System gemäß Anspruch 1, wobei das mindestens eine Polymer ausgewählt ist aus der Gruppe bestehend aus einem Silikon-Acryl-Hybridpolymer, einem Polymer auf Basis von Polysiloxanen, einem Polymer auf Basis von Polyisobutylenen und einem Acrylatpolymer.

3. Transdermales therapeutisches System gemäß Anspruch 1 oder 2, wobei das mindestens eine Polymer in der Fingolimod-enthaltenden Schicht in einer Menge von etwa 40 bis etwa 99 Gew.-%, vorzugsweise von etwa 50 bis etwa 99 Gew.-%, noch bevorzugter von etwa 60 bis etwa 99 Gew.-%, bezogen auf die Fingolimod-enthaltende Schicht, enthalten ist.

4. Das transdermale therapeutische System gemäß einem der Ansprüche 1 bis 3, wobei das mindestens eine Polymer ein Haftkleber auf Polymerbasis ist.

5. Transdermales therapeutisches System gemäß einem der Ansprüche 1 bis 4, wobei die Fingolimod-enthaltende Schicht eine Fingolimod-enthaltende Matrixschicht ist.

6. Transdermales therapeutisches System gemäß einem der Ansprüche 1 bis 5, wobei das Dodecan-1-ol in einer Menge von 2 bis 40 Gew.-%, vorzugsweise von 2 bis 30 Gew.-%, besonders bevorzugt von 4 bis 20 Gew.-%, bezogen auf die Fingolimod-enthaltende Schicht, enthalten ist.

7. Das transdermale therapeutische System gemäß einem der Ansprüche 1 bis 6, wobei das Fingolimod in einer Menge von 1 bis 20 Gew.-%, vorzugsweise von 1 bis 15 Gew.-%, besonders bevorzugt von 2 bis 10 Gew.-%, bezogen auf die Fingolimod-enthaltende Schicht, enthalten ist.

8. Das transdermale therapeutische System gemäß einem der Ansprüche 1 bis 7, wobei die Fingolimod-enthaltende Schichtstruktur 0,1 mg/cm$^2$ bis 2,0 mg/cm$^2$, vorzugsweise 0,1 mg/cm$^2$ bis 1,5 mg/cm$^2$, besonders bevorzugt 0,2 mg/cm$^2$ bis 1,2 mg/cm$^2$ Fingolimod bezogen auf die Fingolimod-enthaltende Schicht enthält.

9. Das transdermale therapeutische System gemäß einem der Ansprüche 1 bis 8, wobei die Fingolimod-enthaltende Schicht erhältlich ist durch Beschichten und Trocknen einer Fingolimod-enthaltenden Beschichtungszusammensetzung, die das mindestens eine Polymer und das Dodecan-1-ol und die therapeutisch wirksame Menge an Fingolimod in einem Gewichtsverhältnis von Dodecan-1-ol: Fingolimod von 1,5 : 1 bis 5 : 1 enthält.

10. Das transdermale therapeutische System gemäß einem der Ansprüche 1 bis 9, wobei die Fingolimod-enthaltende Schicht ferner ein Hilfspolymer umfasst, vorzugsweise ausgewählt aus der Gruppe bestehend aus Alkylmethacrylat-Copolymeren, Aminoalkylmethacrylat-Copolymeren, Methacrylsäure-Copolymeren, Methacrylester-Copolymeren, Ammonioalkylmethacrylat-Copolymeren, Polyvinylpyrrolidonen, Vinylpyrrolidon-Vinylacetat-Copolymeren, Polyvinylcaprolactam-Polyvinylacetat-Polyethylenglykol-Copolymeren, Cellulosederivaten und Mischungen davon, noch bevorzugter ausgewählt aus Cellulosederivaten.

11. Das transdermale therapeutische System gemäß Anspruch 10, wobei das Hilfspolymer in einer Menge von etwa 0,5 bis etwa 20 Gew.-%, vorzugsweise von etwa 0,5 bis etwa 10 Gew.-%, noch bevorzugter von etwa 1 bis etwa 5 Gew.-%, bezogen auf die Fingolimod enthaltende Schicht, enthalten ist.

12. Das transdermale therapeutische System gemäß einem der Ansprüche 1 bis 11, wobei die Fingolimod-enthaltende Schicht kein Polyvinylpyrrolidon enthält.

13. Transdermales therapeutisches System gemäß einem der Ansprüche 1 bis 12, wobei die Fingolimod-enthaltende Schicht keinen Dodecanolester, keine schwefelorganische Verbindung und/oder keinen Fettsäureester enthält.

14. Transdermales therapeutisches System gemäß einem der Ansprüche 1 bis 13, das eine mittlere Freisetzungsrate von Fingolimod von 0,1 bis 1,0 mg/Tag, vorzugsweise über mindestens 72 Stunden, etwa 84 Stunden, etwa 96 Stunden oder etwa 168 Stunden nach Verabreichung, aufweist.

15. Transdermales therapeutisches System gemäß einem der Ansprüche 1 bis 14, das eine kumulative permeierte Menge an Fingolimod von mehr als 1,5 $\mu$g/cm$^2$ innerhalb der ersten 24 Stunden nach der Verabreichung und/oder eine kumulative permeierte Menge an Fingolimod von mehr als 6,0 $\mu$g/cm$^2$ innerhalb der ersten 36 Stunden nach der Verabreichung bereitstellt, gemessen in einer Franz-Diffusionszelle mit dermatisierter menschlicher Haut.

16. Transdermales therapeutisches System gemäß einem der Ansprüche 1 bis 15, das eine Hautpermeationsrate von Fingolimod von mehr als 0,1 $\mu$g/cm$^2$-Stunde in Stunde 16 nach der Verabreichung liefert, gemessen in einer Franz-Diffusionszelle mit dermatomisierter menschlicher Haut.

17. Transdermales therapeutisches System gemäß einem der Ansprüche 1 bis 16, das ein Verhältnis von $C_{max}$ Fingolimodphosphat : $C_{max}$ Fingolimod von 0,2 : 1 bis 0,8 : 1 über einen Zeitraum von etwa 168 Stunden nach Verabreichung einer Einzeldosis an eine Probandengruppe ergibt.

18. Das transdermale therapeutische System gemäß einem der Ansprüche 1 bis 17, zur Verwendung in einem Verfahren zur Behandlung einer Immunkrankheit, vorzugsweise der Multiplen Sklerose.

19. Verfahren zur Herstellung eines transdermalen therapeutischen Systems gemäß einem der Ansprüche 1 bis 18, umfassend die folgenden Schritte:

1) Bereitstellung einer Fingolimod-enthaltenden Beschichtungszusammensetzung, die Folgendes umfasst

a) Fingolimod,
b) mindestens ein Polymer,
c) Dodecan-1-ol, und
d) gegebenenfalls ein Lösungsmittel,

2) Auftragen der Fingolimod-enthaltenden Beschichtungszusammensetzung auf eine Trennfolie in einer Menge, die das gewünschte Flächengewicht ergibt,

3) Trocknen der beschichteten Fingolimod-enthaltenden Beschichtungszusammensetzung, um die Fingolimod-enthaltenden Schicht bereitzustellen,

4) Laminieren der Fingolimod-enthaltenden Schicht auf eine Rückschicht, um eine Fingolimod-enthaltende Schichtstruktur zu erhalten,

5) gegebenenfalls Bereitstellen einer zusätzlichen Hautkontaktschicht durch Beschichten und Trocknen einer wirkstofffreien Beschichtungszusammensetzung oder einer wirkstoffhaltigen Beschichtungszusammensetzung gemäß den Schritten 2 und 3, Entfernen der Trennfolie der Fingolimod-enthaltenden Schicht und Laminieren der Klebeseite der Hautkontaktschicht auf die Klebeseite der Fingolimod-enthaltenden Schicht, um eine Fingolimod-enthaltende Schichtstruktur bereitzustellen,

6) Ausstanzen der einzelnen Systeme aus der Fingolimod-enthaltenden Schichtstruktur,

7) gegebenenfalls Aufkleben einer wirkstofffreien selbstklebenden Schichtstruktur auf die einzelnen Systeme, die auch eine Rückschicht und eine wirkstofffreie Haftklebeschicht umfasst und größer ist als die einzelnen Systeme der Fingolimod-enthaltenden Schichtstruktur.

20. Verwendung von Dodecan-1-ol in einem Fingolimod-enthaltenden transdermalen therapeutischen System zur Verkürzung der Verzögerungszeit der Permeation von Fingolimod.

## Revendications

1. Un système thérapeutique transdermique pour l'administration transdermique de fingolimod comprenant une structure de couches contenant du fingolimod,

   la structure de couches contenant du fingolimod comprenant:

   A) une couche support, et
   B) une couche contenant du fingolimod comprenant:

   a) une quantité thérapeutiquement efficace de fingolimod
   b) au moins un polymère, et
   c) du dodécan-1-ol, dans lequel le rapport pondéral dodécan-1-ol/fingolimod est compris entre 1,5/1 et 5/1.

2. Le système thérapeutique transdermique selon la revendication 1, dans lequel le au moins un polymère est sélectionné dans le groupe consistant en un polymère hybride silicone acrylique, un polymère à base de polysiloxanes, un polymère à base de polyisobutylènes et un polymère d'acrylate.

3. Le système thérapeutique transdermique selon la revendication 1 ou 2, dans lequel le au moins un polymère est contenu dans la couche contenant le fingolimod en une quantité comprise entre environ 40% et environ 99% en poids, de préférence entre environ 50% et environ 99% en poids, plus préférablement entre environ 60% et environ 99% en poids exprimés par rapport à la couche contenant le fingolimod.

4. Le système thérapeutique transdermique selon une quelconque des revendications 1 à 3, dans lequel le au moins un polymère est un adhésif sensible à la pression à base de polymère.

5. Le système thérapeutique transdermique selon une quelconque des revendications 1 à 4, dans lequel la couche contenant le fingolimod est une couche matricielle contenant le fingolimod.

6. Le système thérapeutique transdermique selon une quelconque des revendications 1 à 5, dans lequel le dodécan-l-ol est contenu en une quantité comprise entre 2% et 40% en poids, préférablement entre 2% et 30% en poids, plus préférablement entre 4% et 20% en poids exprimé par rapport à la couche contenant le fingolimod.

7. Le système thérapeutique transdermique selon une quelconque des revendications 1 à 6, dans lequel le fingolimod est contenu en une quantité comprise entre 1 % et 20% en poids, préférablement entre 1 % et 15% en poids, plus préférablement entre 2% et 10% en poids exprimés par rapport à la couche contenant le fingolimod.

8. Le système thérapeutique transdermique selon une quelconque des revendications 1 à 7, dans lequel la structure de couches contenant le fingolimod contient de 0,1 mg/cm$^2$ à 2,0 mg/cm$^2$, préférablement 0,1 mg/cm$^2$ à 1,5 mg/cm$^2$,

plus préférablement 0,2 mg/cm$^2$ à 1,2 mg/cm$^2$ de fingolimod exprimés par rapport à la couche contenant le fingolimod.

9. Le système thérapeutique transdermique selon une quelconque des revendications 1 à 8, dans lequel la couche contenant le fingolimod est susceptible d'être obtenue par revêtement et séchage d'une composition de revêtement contenant le fingolimod, qui comprend le au moins un polymère et le dodécan-1-ol et la quantité thérapeutiquement efficace de fingolimod en un rapport pondéral dodécan-1-ol/fingolimod compris entre 1,5/1 et 5/1.

10. Le système thérapeutique transdermique selon une quelconque des revendications 1 à 9, dans lequel la couche contenant le fingolimod comprend en outre un polymère auxiliaire, de préférence sélectionné dans le groupe consistant en copolymères d'alkyl méthacrylate, copolymères d'amino alkyl méthacrylate, copolymères d'acide méthacrylique, copolymères d'ester méthacrylique, copolymères d'ammonioalkyl-méthacrylate, polyvinylpyrrolidones, copolymères de vinylpyrrolidone-acétate de vinyl, copolymères de polyvinylcaprolactame-polivinyleacétate-polyéthylène glycol, dérivés de cellulose, et les mélanges en dérivant, plus préférablement sélectionnés parmi les dérivés de cellulose.

11. Le système thérapeutique transdermique selon la revendication 10, dans lequel le polymère auxiliaire est contenu en une quantité comprise entre environ 0,5% et environ 20%, préférablement d'environ 0,5% à environ 10% en poids, plus préférablement d'environ 1% à environ 5% en poids, en poids exprimé par rapport à la couche contenant le fingolimod.

12. Le système thérapeutique transdermique selon l'une quelconque des revendications 1 à 11, dans lequel la couche contenant le fingolimod ne comprend pas de polyvinylpyrrolidone.

13. Le système thérapeutique transdermique selon l'une quelconque des revendications 1 à 12, dans lequel la couche contenant le fingolimod ne comprend pas d'ester de dodécanol, de composé organosulfuré et/ou d'ester d'acide gras.

14. Le système thérapeutique transdermique selon l'une quelconque des revendications 1 à 13, qui conduit à un taux moyen de libération de fingolimod de 0,1 à 1,0 mg/jour, préférablement sur au moins 72 heures, environ 84 heures, environ 96 heures ou environ 168 heures, après l'administration.

15. Le système thérapeutique transdermique selon une quelconque des revendications 1 à 14, qui fournit dans les premières 24 heures après administration, un taux de pénétration cumulatif de fingolimod supérieur à 1,5 $\mu$g/cm$^2$ et/ou dans les premières 36 heures après administration, un taux de pénétration cumulatif de fingolimod supérieur à 6,0 $\mu$g/cm$^2$, tel que mesurés dans une cellule de diffusion de Franz sur une peau humaine dermatomée.

16. Le système thérapeutique transdermique selon une quelconque des revendications 1 à 15, qui conduit 16 heures après administration, à l'obtention d'un taux d'absorption cutanée de fingolimod supérieur à 0,1 $\mu$g/cm$^2$-hr, tel que mesuré dans une cellule de diffusion de Franz sur une peau humaine dermatomée.

17. Le système thérapeutique transdermique selon une quelconque des revendications 1 à 16, qui fournit un rapport $C_{max}$ de phosphate de fingolimod / $C_{max}$ de fingolimod de 0,2/1 à 0,8/1 sur environ 168 heures d'administration après une dose unique d'administration à une population de patients.

18. Le système thérapeutique transdermique selon une quelconque des revendications 1 à 17, pour une utilisation dans un procédé de traitement d'un trouble immunitaire, préférablement de la sclérose en plaques.

19. Un procédé de fabrication d'un système thérapeutique transdermique selon une quelconque des revendications 1 à 18, comprenant les étapes de :

    1) fourniture d'une composition de revêtement contenant du fingolimod comprenant :

        a) fingolimod
        b) au moins un polymère,
        c) dodécan-I-ol, et
        d) éventuellement un solvant,

    2) application de la composition de revêtement contenant du fingolimod sur un film de séparation en une quantité permettant d'obtenir le poids surfacique souhaité,

3) le séchage de la composition de revêtement contenant le fingolimod revêtu pour obtenir une couche contenant le fingolimod,

4) le laminage de la couche contenant le fingolimod sur une couche support pour obtenir une couche contenant le fingolimod,

5) éventuellement la fourniture d'une couche supplémentaire de contact avec la peau par enduction et séchage d'une composition de revêtement exempte d'agent actif ou d'une composition de revêtement contenant l'agent actif selon les étapes 2 et 3, et le retrait du film de séparation de la couche contenant du fingolimod et le laminage de la côté adhésif de la couche de contact avec la peau sur la côté adhésif de la couche contenant le fingolimod pour obtenir une structure de couche contenant le fingolimod,

6) le poinçonnage des systèmes individuels de la structure de couches contenant du fingolimod.

7) éventuellement le collage aux systèmes individuels d'une structure de couches auto-adhésives exemptes d'agent actif comprenant également une couche de support et une couche adhésive sensible à la pression, exempte d'agent actif, et qui est plus grande que les systèmes individuels de la structure de couches contenant du fingolimod.

20. Utilisation de dodécan-1-ol d'un système thérapeutique transdermique comprenant du fingolimod pour réduire le temps de latence lors de la perméation du fingolimod.

Fig. 1a

Fig. 1b

**Fig. 2a**

**Fig. 2b**

**Fig. 3a**

**Fig. 3b**

**Fig. 4a**

**Fig. 4b**

**Fig. 5a**

**Fig. 5b**

**Fig. 6**

**Fig. 7**

**Fig. 8**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018028461 A1
- EP 2599847 A
- WO 2016130408 A
- WO 2007145996 A
- EP 2599847 A1
- WO 2010124187 A